# EUROPEAN PATENT APPLICATION

(11) **EP 4 523 714 A1**
(43) Date of publication of application: **19.03.2025**
(21) Application number: 23857209.3
(22) Date of filing: 09.08.2023
(51) Int. Cl.: A61L 27/34, A61L 29/08, A61L 31/10, C08F 290/06, C08J 7/04, G02C 7/04

(54) **COATED MEDICAL DEVICE AND METHOD FOR MANUFACTURING SAME**

(30) Priority: 26.08.2022 JP 2022134677; 28.02.2023 JP 2023029448
(71) Applicant: Toray Industries, Inc., Tokyo 103-8666 (JP)
(72) Inventor: KITAGAWA Rumiko, Otsu-shi, Shiga 520-8558 (JP)
(74) Representative: Prüfer & Partner mbB Patentanwälte · Rechtsanwälte
(86) International application number: PCT/JP2023/029077
(87) International publication number: WO 2024/043096

(57) **Abstract**

An object of the present invention is to provide a coated medical device allowed to have not only sufficient hydrophilicity and lubricity but also a lipid adhesion inhibiting capability, and to provide a simple method of manufacturing the device. The present invention provides a coated medical device and a method of manufacturing the same, the coated medical device including a medical device and a hydrophilic polymer layer coating the surface of the medical device; wherein the hydrophilic polymer layer contains a hydrophilic polymer A, the hydrophilic polymer A containing, as monomer units, a compound a1 having a specific structure and a compound a2 having an amide group; and wherein the copolymerization ratio of the compound a1 to the compound a2 is 1/99 to 90/10 by mass.

## Description

### Technical Field

The present invention relates to a coated medical device and a method of manufacturing the same.

### BACKGROUND ART

Medical devices produced using soft materials such as a silicone rubber and hydrogel or medical devices produced using hard materials such as metal and glass have hitherto been used for diverse applications in various fields.

In cases where a medical device is introduced into a living body or attached to the surface of a living body, it becomes important to modify the surface of the medical device for the purpose of enhancing the biocompatibility of the device. If the surface modification not only enhances the biocompatibility but also allows the medical device to have properties such as hydrophilicity, lubricity, and a lipid adhesion inhibiting capability, users can expect an improvement in feeling of use, a reduction in discomfort, an improvement in symptoms, and the like.

Examples of a known method of modifying the surface of a medical device include a method in which a medical device is immersed at room temperature in a pH 6 to 9 solution containing one or more polymers including a compound having an amide group such as N,N-dimethylacrylamide or vinylpyrrolidone, or heated after immersion, thereby modifying the surface of the medical device (Patent Literature 1 to 3).

### Citation List

### Patent Literature

Patent Literature 1: WO 2017/018425
Patent Literature 2: WO 2015/119256
Patent Literature 3: JP 5154231

### Summary of Invention

### Technical Problem

However, none of the inventions disclosed in Patent Literatures 1 to 3 can afford sufficient hydrophilicity, lubricity, or the like to the surface of a medical device, and furthermore, is a method that can afford a lipid adhesion inhibiting capability to a medical device.

In view of this, an object of the present invention is to provide a coated medical device allowed to have not only sufficient hydrophilicity and lubricity but also a lipid adhesion inhibiting capability, and to provide a simple method of manufacturing the device.

### Solution to Problem

To achieve the above-described object, the present invention is a coated medical device including: a medical device and a hydrophilic polymer layer coating the surface of the medical device; wherein the hydrophilic polymer layer contains a hydrophilic polymer A, the hydrophilic polymer A containing, as monomer units, a compound a1 of the following general formula (I) or the following general formula (II) and a compound a2 having an amide group; and wherein the copolymerization ratio of the compound a1 to the compound a2 is 1/99 to 90/10 by mass. 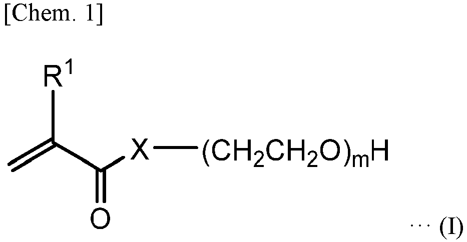 wherein, in the general formula (I), R¹ is a hydrogen atom or a methyl group; X is an oxygen atom or NR², wherein R² is a hydrogen atom or an alkyl group; and m is an integer of 1 to 30. 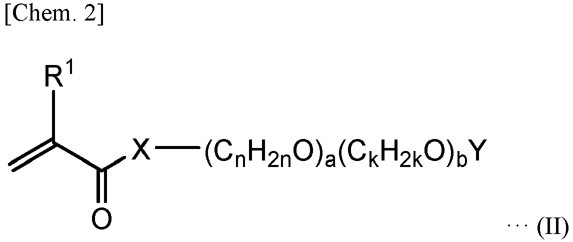 wherein, in the general formula (II), R¹ is a hydrogen atom or a methyl group; X is an oxygen atom or NR², wherein R² is a hydrogen atom or an alkyl group; n is 2 or 4; k is 3 or 4; a is an integer of 0 to 30; b is an integer of 1 to 30; and Y is a hydrogen atom, a methyl group, an ethyl group, a propyl group, an isopropyl group, or a butyl group.

In addition, the present invention is a method of manufacturing the coated medical device, including: (A) a contacting step of housing a medical device in a container, and bringing the medical device into contact with a solution a containing a hydrophilic polymer A; and (C) a heating step of heating the container; wherein the hydrophilic polymer A contains, as monomer units, a compound a1 of the above-described general formula (I) or the above-described general formula (II) and a compound a2 having an amide group; wherein the copolymerization ratio of the compound a1 to the compound a2 is 1/99 to 90/10 by mass; and
wherein, in the general formula (I), R¹ is a hydrogen atom or a methyl group; X is an oxygen atom or NR², wherein R² is a hydrogen atom or an alkyl group; and m is an integer of 1 to 30;
wherein, in the general formula (II), R¹ is a hydrogen atom or a methyl group; X is an oxygen atom or NR², wherein R² is a hydrogen atom or an alkyl group; n is 2 or 4; k is 3 or 4; a is an integer of 0 to 30; b is an integer of 1 to 30; and Y is a hydrogen atom, a methyl group, an ethyl group, a propyl group, an isopropyl group, or a butyl group; and
wherein the pH of the solution a after the above-described heating step is 6.1 to 8.0.

### Advantageous Effects of Invention

The present invention makes it possible to provide a coated medical device allowed to have not only sufficient hydrophilicity and lubricity demanded for a medical device but also a lipid adhesion inhibiting capability. In addition, the manufacturing method according to the present invention makes it possible to obtain a coated medical device in a simple process.

### Description of Embodiments

A coated medical device according to the present invention includes a medical device and a hydrophilic polymer layer coating the surface of the medical device.

Examples of the medical device included in the coated medical device according to the present invention include an ophthalmic lens, a dermal covering material, a wound dressing material, a skin protection material, a skin medicine carrier, an infusion tube, a gas delivery tube, a drainage tube, a blood circuit, a covering tube, a catheter, a stent, a sheath, a biosensor chip, an artificial heart and lung, or an endoscopic covering material. Here, examples of the ophthalmic lens include a contact lens, an intraocular lens, an artificial cornea, a corneal inlay, a corneal onlay, and an eyeglass lens.

A material constituting the medical device may be any one of a hydrous material and a low water content material. Examples of the hydrous material include a hydrogel and a silicone hydrogel. In cases where the medical device is a contact lens, a hydrogel is preferable because the hydrogel can form a durable surface layer having a high lipid adhesion inhibiting capability, and exhibiting excellent hydrophilicity and lubricity, and sustain the effect during long-time attachment. Examples of the low water content material include a low water content soft material and a low water content hard material. In this regard, the low water content material refers to a material the moisture content of which is 10% by mass or less.

Hereinafter, United States Adopted Names (USAN) is used to express the name of a hydrogel or a silicone hydrogel in some cases. In the USAN, variations of a material are expressed with a symbol such as A, B, or C added at the end in some cases, and in the present specification, a name having no symbol added at the end represents all the variations. For example, a name expressed simply as "ocufilcon" represents all variations of ocufilcon, such as "ocufilcon A", "ocufilcon B", "ocufilcon C", "ocufilcon D", "ocufilcon E", and "ocufilcon F".

Examples of the hydrogel include tefilcon, tetrafilcon, hefilcon, mafilcon, polymacon, hioxifilcon, alfafilcon, omafilcon, hixoifilcon, nelfilcon, nesofilcon, hilafilcon, acofilcon, deltafilcon, etafilcon, focofilcon, ocufilcon, phemfilcon, methafilcon, and vilfilcon.

A medical device that is a contact lens composed of a hydrogel belongs to Group 1 to Group 4 prescribed in the classification of contact lenses by the Food and Drug Administration (FDA). Group 2 or Group 4 that exhibits good hydrophilicity is preferable, and Group 4 is more preferable.

Examples of a nonionic hydrogel which belongs to Group 1 and the moisture content of which is less than 50% by mass include tefilcon, tetrafilcon, helfilcon, mafilcon, polymacon, and hioxifilcon.

Examples of a nonionic hydrogel which belongs to Group 2 and the moisture content of which is 50% by mass or more include alfafilcon, omafilcon, hixoifilcon, nelfilcon, nesofilcon, hilafilcon, and acofilcon. Omafilcon, hixoifilcon, nelfilcon, or nesofilcon, which each exhibits good hydrophilicity, is preferable. Omafilcon or hixoifilcon is more preferable. Omafilcon is still more preferable.

Examples of an ionic hydrogel which belongs to Group 3 and the moisture content of which is less than 50% by mass include deltafilcon.

Examples of an ionic hydrogel which belongs to Group 4 and the moisture content of which is 50% by mass or more include etafilcon, focofilcon, ocufilcon, phemfilcon, methafilcon, and vilfilcon. Etafilcon, focofilcon, ocufilcon, or phemfilcon, which each exhibits good hydrophilicity, is preferable. Etafilcon or ocufilcon is more preferable, and etafilcon is still more preferable.

A medical device that is a contact lens composed of a silicone hydrogel preferably belongs to Group 5 prescribed in the classification of contact lenses by the Food and Drug Administration (FDA).

A silicone hydrogel belonging to Group 5 is preferably a polymer having a silicon atom in the main chain and/or side chain, and having hydrophilicity, and examples thereof include a copolymer of a monomer having a siloxane bond and a hydrophilic monomer. Examples of such a copolymer include lotrafilcon, galyfilcon, narafilcon, senofilcon, comfilcon, enfilcon, balafilcon, efrofilcon, fanfilcon, somofilcon, samfilcon, olifilcon, asmofilcon, formofilcon, stenfilcon, abafilcon, mangofilcon, riofilcon, sifilcon, larafilcon, and delefilcon. Lotrafilcon, galyfilcon, narafilcon, senofilcon, comfilcon, enfilcon, stenfilcon, somofilcon, delefilcon, balafilcon, or samfilcon, which each exhibits not only a lipid adhesion inhibiting capability but also good hydrophilicity and antifouling properties, is preferable. Lotrafilcon, narafilcon, senofilcon, comfilcon, or enfilcon is more preferable. Narafilcon, senofilcon, or comfilcon is still more preferable.

In cases where the medical device is a contact lens, the low water content soft material or the low water content hard material is preferably a silicon atomcontaining material that exhibits a high oxygen permeability, and is capable of supplying sufficient oxygen to the cornea.

The low water content hard material is preferably a low water content hard material that belongs to the classification of contact lens prescribed by the Food and Drug Administration (FDA).

The low water content hard material is preferably a polymer having, in the main chain and/or a side chain, a silicon atom such as in a siloxane bond, more preferably a material having a high oxygen permeability, for example, tris(trimethylsilyloxy)silyl]propyl methacrylate, polydimethyl siloxane having a double bond at both ends, a homopolymer containing a silicone-containing acrylate, silicone-containing methacrylate, or the like, or a copolymer of such a polymer and another monomer.

Specifically, the above-described low water content hard material is preferably selected from the group consisting of neofocon, pasifocon, telefocon, silafocon, paflufocon, petrafocon, and fluorofocon. Among these, neofocon, pasifocon, telefocon, or silafocon is more preferable from the viewpoint of exhibiting a good lipid adhesion inhibiting capability and exhibiting antifouling properties. Neofocon, pasifocon, or telefocon is still more preferable. Neofocon is particularly preferable.

**In** cases where the medical device is other than a contact lens, the low water content hard material is preferably polyethylene, polypropylene, polysulfone, polyetherimide, polystyrene, polymethyl methacrylate, polyamide, polyester, epoxy resin, polyurethane, or polyvinyl chloride. Among these, polysulfone, polystyrene, polymethyl methacrylate, polyurethane, or polyamide is more preferable from the viewpoint of exhibiting a good lipid adhesion inhibiting capability and exhibiting antifouling properties. Polymethyl methacrylate is still more preferable.

Examples of the low water content soft material include: a material disclosed in WO2013/024799, and having a moisture content of 10% by mass or less, an elastic modulus of 100 to 2,000 kPa, and a tensile elongation of 50 to 3,000%; and elastofilcon.

Whether the medical device is hydrous or has a low water content in the present invention, the surface of the medical device is enabled to have a suitable lipid adhesion inhibiting capability. **In** order to afford a suitable lipid adhesion inhibiting capability, the moisture content of the medical device is preferably 0.0001% by mass or more, more preferably 0.001% by mass or more. **In** addition, the moisture content of the medical device is preferably 80% by mass or less, more preferably 70% by mass or less, still more preferably 60% by mass or less.

In order that a medical device that is a contact lens can make it easy to ensure the movement of the lens in an eye, the moisture content of the medical device is preferably 15% by mass or more, more preferably 20% by mass or more.

The hydrophilic polymer A is hydrophilic. Here, being "hydrophilic" means that the polymer in an amount of 0.0001 part by mass or more is soluble in 100 parts by mass of water or a liquid mixture of 100 parts by mass of water and 100 parts by mass of tert-butanol at room temperature (20 to 23°C). The hydrophilic polymer is preferably soluble in an amount of 0.01 part by mass or more, more preferably 0.1 part by mass or more, still more preferably 1 part by mass or more.

The hydrophilic polymer layer included in the coated medical device according to the present invention is characterized by containing a hydrophilic polymer A, the hydrophilic polymer A containing, as monomer units, a compound a1 of the following general formula (I) or the following general formula (II) and a compound a2 having an amide group, wherein the copolymerization ratio of the compound a1 to the compound a2 is 1/99 to 90/10 by mass.

A compound that can be used as each of the compound a1 and the compound a2 is a single monomer or a plurality of different monomers having different structures.

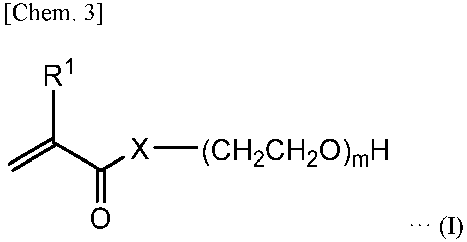

In the general formula (I), R¹ is a hydrogen atom or a methyl group; X is an oxygen atom or NR², wherein R² is a hydrogen atom or an alkyl group; and m is an integer of 1 to 30.

R² that is an alkyl group may be linear or branched, and is preferably a C1-10 alkyl group. Examples of R² include a methyl group, ethyl group, propyl group, 2-propyl group, butyl group, 2-butyl group, tert-butyl group, pentyl group, 2-pentyl group, 3-pentyl group, hexyl group, heptyl group, and octyl group.

m is preferably 2 to 30 from the viewpoint of suitable hydrophilicity and easy polymerization. The lower limit of m is more preferably 3, still more preferably 4, particularly preferably 5. The upper limit of m is more preferably 25, still more preferably 20, particularly preferably 10.

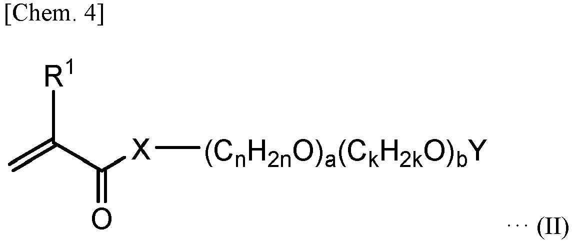

In the general formula (II), R¹ is a hydrogen atom or a methyl group; X is an oxygen atom or NR², wherein R² is a hydrogen atom or an alkyl group; n is 2 or 4; k is 3 or 4; a is an integer of 0 to 30; b is an integer of 1 to 30; and Y is a hydrogen atom, a methyl group, an ethyl group, a propyl group, an isopropyl group, or a butyl group.

R² that is an alkyl group may be linear or branched, and is preferably a C1-10 alkyl group. Examples of R² include a methyl group, ethyl group, propyl group, 2-propyl group, butyl group, 2-butyl group, tert-butyl group, pentyl group, 2-pentyl group, 3-pentyl group, hexyl group, heptyl group, and octyl group.

a is preferably 0 to 25 from the viewpoint of suitable hydrophilicity and easy polymerization. The lower limit of a is more preferably 0, still more preferably 2, particularly preferably 3. The upper limit of a is more preferably 20, still more preferably 15, particularly preferably 10.

b is preferably 1 to 25 from the viewpoint of suitable hydrophilicity and easy polymerization. The lower limit of b is more preferably 1, still more preferably 2, particularly preferably 3. The upper limit of b is more preferably 20, still more preferably 15, particularly preferably 10.

Y is more preferably hydrogen, a methyl group, ethyl group, propyl group, or isopropyl group, most preferably hydrogen or a methyl group, from the viewpoint of easy formation of a coating layer.

The compound a1 is more preferably a compound of the general formula (I), from the viewpoint of easy polymerization and ease of imparting solubility in a water-soluble solvent and hydrophilicity.

The compound a2 having an amide group is preferably a compound having an acrylamide group or a methacrylamide group, or an N-vinyl carboxylic amide (encompassing a cyclic compound) from the viewpoint of easy polymerization.

Specific examples include N-vinylpyrrolidone, N-vinylcaprolactam, N-vinylacetamide, N-methyl-N-vinylacetamide, N-vinylformamide, N,N-dimethylacrylamide, N-isopropylacrylamide, N-methylacrylamide, N-ethylacrylamide, N-butylacrylamide, N-tert-butylacrylamide, N-hydroxymethylacrylamide, N-methoxymethylacrylamide, N-ethoxymethylacrylamide, N-propoxymethylacrylamide, N-isopropoxymethylacrylamide, N-(2-hydroxyethyl)acrylamide, N-butoxymethylacrylamide, N-isobutoxymethylacrylamide, N-hydroxymethylmethacrylamide, N-methoxymethylmethacrylamide, N-ethoxymethylmethacrylamide, N-propoxymethylmethacrylamide, N-butoxymethylmethacrylamide, N-isobutoxymethylmethacrylamide, acryloyl morpholine, and acrylamide. For the purpose of enhancing the lubricity, N-vinylpyrrolidone, N-isopropylacrylamide, or N,N-dimethylacrylamide is preferable. N-isopropylacrylamide, or N,N-dimethylacrylamide is more preferable, and N,N-dimethylacrylamide is still more preferable.

The hydrophilic polymer A may contain, as a monomer unit, another compound other than the compound a1 of the general formula (I) or the general formula (II) and the compound a2 having an amide group, in the form of copolymer of one or more than one of the compounds. Examples of such a compound include glycerol acrylate, glycerol methacrylate, N-(4-hydroxyphenyl)maleimide, hydroxystyrene, a vinyl alcohol (a vinyl carboxylate ester as a precursor), hydroxyethyl (meth)acrylate, hydroxypropyl (meth)acrylate, and hydroxybutyl (meth)acrylate. To enhance the antifouling properties for body fluids, glycerol acrylate, glycerol methacrylate, or a vinyl alcohol is preferable, and glycerol acrylate or glycerol methacrylate is more preferable. In addition, it is also possible to use, as the another compound, a compound having functions such as hydrophilicity, antibacterial properties, antifouling properties, and medicinal effects.

Because the hydrophilic polymer A contains, as a monomer unit, the compound a1 having a specific structure, the hydrophilic polymer A is easily dissolved in water, exhibits a hydrophilic function, and can form a surface that has not only hydrophilicity but also a lipid adhesion inhibiting capability.

Because the hydrophilic polymer A contains, as a monomer unit, the compound a2 having an amide group, the hydrophilic polymer A exhibits a suitable viscosity when dissolved in water, and thus, can form a surface that is not only hydrophilic but also lubricious.

The copolymerization ratio of the compound a1 to the compound a2 is in the range of from 1/99 to 90/10 by mass to make it easier to exhibit a function such as a lipid adhesion inhibiting capability or durable hydrophilicity. That is, with respect to 100% by mass of the total of the compound a1 and the compound a2 among the whole monomer units included in the hydrophilic polymer A, the ratio of the compound a1 needs to be 1% by mass or more, and is preferably 10% by mass or more, more preferably 30% by mass or more. In addition, the ratio of the compound a1 needs to be 99% by mass or less, and is preferably 90% by mass or less, more preferably 85% by mass or less, still more preferably 80% by mass or less, from the viewpoint of easy polymerization. In addition, with respect to 100% by mass of the whole hydrophilic polymer A, the ratio of the compound a2 needs to be 10% by mass or more, and is preferably 12% by mass or more, more preferably 15% by mass or more, still more preferably 20% by mass or more. In addition, the ratio of the compound a2 needs to be 90% by mass or less, and is preferably 90% by mass or less, more preferably 80% by mass or less, still more preferably 70% by mass or less.

When other compound other than the above compounds a1 and a2 are copolymerized as a third monomer component, the copolymerization ratio of the third monomer component is more preferably 2% by mass or more, still more preferably 5% by mass or more, and particularly preferably 10% by mass or more. In addition, the copolymerization ratio of the third monomer component is more preferably 90% by mass or less, still more preferably 80% by mass or less, particularly preferably 70% by mass or less.

When the copolymerization ratio of the compound a1 of the above general formula (I), the compound a2 having an amide group, and the third monomer component is within the above range, it is easier to exhibit functions such as lubricity and the antifouling properties for body fluids.

To enhance the adsorptive force of the hydrophilic polymer A on the surface of the medical device, and provide the medical device with a sufficient lipid adhesion inhibiting capability, the weight-average molecular weight of the hydrophilic polymer A is preferably 2,000 to 1,500,000. The weight-average molecular weight is more preferably 5,000 or more, still more preferably 10,000 or more, particularly preferably 100,000 or more. **In** addition, the weight-average molecular weight is more preferably 1,200,000 or less, still more preferably 1,000,000 or less, particularly preferably 900,000 or less. Here, the weight-average molecular weight is a weight-average molecular weight in terms of polyethylene glycol, as measured by a gel permeation chromatography method in which an aqueous solvent is used as an eluent.

In addition, a method of manufacturing a coated medical device according to the present invention includes: (A) a contacting step of housing a medical device in a container, and bringing the medical device into contact with a solution a containing a hydrophilic polymer A; and (C) a heating step of heating the container; wherein the hydrophilic polymer A contains, as monomer units, a compound a1 of the following general formula (I) or the following general formula (II) and a compound a2 having an amide group; wherein the copolymerization ratio of the compound a1 to the compound a2 is 1/99 to 90/10 by mass; 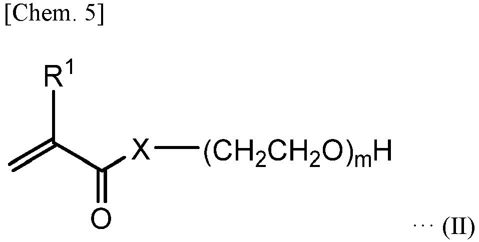
wherein, in the general formula (I), R¹ is a hydrogen atom or a methyl group; X is an oxygen atom or NR², wherein R² is a hydrogen atom or an alkyl group; and m is an integer of 1 to 30;

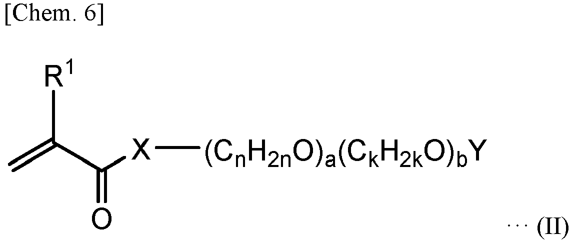

wherein, in the general formula (II), R¹ is a hydrogen atom or a methyl group; X is an oxygen atom or NR², wherein R² is a hydrogen atom or an alkyl group; n is 2 or 4; k is 3 or 4; a is an integer of 0 to 30; b is an integer of 1 to 30; and Y is a hydrogen atom, a methyl group, an ethyl group, a propyl group, an isopropyl group, or a butyl group; and
wherein the pH of the solution a after the heating step is 6.1 to 8.0.

The concentration of the hydrophilic polymer A in the solution a containing the hydrophilic polymer A is preferably in the range of from 0.0001 to 30% by mass to adjust the viscosity of the solution a suitably. The concentration of the hydrophilic polymer A is more preferably 0.001% by mass or more, still more preferably 0.005% by mass or more. In addition, the concentration of the hydrophilic polymer A is more preferably 10.0% by mass or less, still more preferably 5.0% by mass or less, still more preferably 1.0% by mass or less, particularly preferably 0.5% by mass or less.

A solvent of the solution a containing the hydrophilic polymer A is preferably a water-soluble organic solvent or water, or a solvent mixture thereof from the viewpoint of easy handling. A solvent mixture of water and a water-soluble organic solvent, or water is more preferable, and water is still more preferable. The water-soluble organic solvent is preferably a water-soluble alcohol, more preferably a water-soluble alcohol having 6 or less carbon atoms, still more preferably a water-soluble alcohol having 5 or less carbon atoms. The solution a may further contain a buffering agent or another additive.

Examples of the buffering agent to be contained in the solution a include boric acid, borates (for example, sodium borate), citric acid, citrates (for example, potassium citrate), bicarbonate (for example, sodium bicarbonate), phosphate buffer solution (for example, Na₂HPO₄, NaH₂PO₄, and KH₂PO₄), TRIS (tris(hydroxymethyl)aminomethane), 2-bis(2-hydroxyethyl)amino-2-(hydroxymethyl)-1,3-propanediol, bis-aminopolyol, triethanolamine, ACES (N-(2-acetamide)-2-aminoethanesulfonic acid), BES (N,N-bis(2-hydroxyethyl)-2-aminoethanesulfonic acid), HEPES (4-(2-hydroxyethyl)-1-piperazineethanesulfonic acid), MES (2-(N-morpholino)ethanesulfonic acid), MOPS (3-[N-morpholino]-propanesulfonic acid), PIPES (piperazine-N,N'-bis(2-ethanesulfonic acid)), TES (N-[tris(hydroxymethyl)methyl]-2-aminoethanesulfonic acid), and salts thereof. The ratio of the buffering agent in the solution a can be suitably adjusted to achieve a desired pH, and usually, the ratio is preferably 0.001 to 2.000% by mass. The ratio of the buffering agent is more preferably 0.010% by mass or more, still more preferably 0.050% by mass or more. In addition, the ratio of the buffering agent is more preferably 1.000% by mass or less, still more preferably 0.300% by mass or less.

The solution a to be subjected to the contacting step is preferably prepared by dissolving the hydrophilic polymer A in a buffering agent solution containing a solvent and a buffering agent. The pH of the buffering agent solution to be used for adjustment of the solution a is preferably within a physiologically acceptable range of from 6.3 to 7.8. The pH of the buffering agent solution is more preferably 6.5 or more, still more preferably 6.8 or more. In addition, the pH of the buffering agent solution is more preferably 7.6 or less, still more preferably 7.4 or less.

In cases where the medical device is an ophthalmic lens, examples of the container for housing the medical device in the contacting step include a vial or blister container to be used for packaging an ophthalmic lens. A blister container is usually composed of: a plastic base portion surrounded by a flat flange standing in a warped manner around the brim of the cavity of the container; and a soft cover sheet bonded to the flat flange and configured to hermetically seal the cavity.

Examples of the material for the plastic base portion include fluorine resins, polyamides, polyacrylate, polyethylene, nylons, olefin copolymers (for example, a polypropylene polyethylene copolymer), polyethylene terephthalate, polyvinyl chloride, amorphous polyolefins, polycarbonate, polysulfone, polybutylene terephthalate, polypropylene, polymethyl pentene, polyesters, rubbers, and urethane.

Examples of the soft cover sheet include a laminate material such as a polypropylene sheet coated with an aluminum foil.

A method of manufacturing a coated medical device according to the present invention includes a heating step of heating (C) the above-described container. In the heating step (C), the medical device is heated together with the container in a state where the medical device is in contact with a solution a containing a hydrophilic polymer A.

Examples of the above-described heating method include a heating method (with hot air), high-pressure steam sterilization method, dry heat sterilization method, burning sterilization method, boiling sterilization method, free-flowing steam sterilization method, electromagnetic wave (γ-ray, microwave, or the like) irradiation, ethylene oxide gas sterilization method (EOG sterilization method), or ultraviolet sterilization method. A high-pressure steam sterilization method is preferable from the viewpoint of the capability to afford a sufficient lipid adhesion inhibiting capability to the medical device and of the low manufacturing cost. Autoclave sterilization performed using a device that is an autoclave is more preferable.

The heating temperature is preferably 80 to 200°C to provide the medical device with a sufficient lipid adhesion inhibiting capability, and simultaneously to cause no influence on the strength of the resulting coated medical device itself. The heating temperature is more preferably 90°C or more, still more preferably 105°C or more, still more preferably 110°C or more, still more preferably 115°C or more, particularly preferably 121°C or more. In addition, the heating temperature is more preferably 180°C or less, still more preferably 170°C or less, particularly preferably 150°C or less.

In addition, the heating time is preferably 1 to 600 minutes for the same reason as with the heating temperature. The heating time is more preferably 2 minutes or more, still more preferably 5 minutes or more, particularly preferably 10 minutes or more. In addition, the heating time is more preferably 400 minutes or less, still more preferably 300 minutes or less, particularly preferably 100 minutes or less.

A method of manufacturing a coated medical device according to the present invention preferably further includes (B) a hermetical sealing step in which the container with the medical device housed therein is hermetically sealed after the above-described contacting step and before the heating step. Hermetically sealing the container with the medical device housed therein, followed by performing the heating step, provides the surface of the medical device with a lipid adhesion inhibiting capability, simultaneously sterilizes the resulting coated medical device, makes it possible to maintain the sterilized state, and hence, is advantageous from the viewpoint of reducing the manufacturing processes. That is, it is preferable in a method of manufacturing a coated medical device according to the present invention that the hermetical sealing step is included and the medical device is sterilized by the heating step to simplify the manufacturing processes. Here, examples of a means for hermetically sealing the container include a means for hermetically sealing the container using a vial with a cap or a blister container as a container for housing the medical device. In addition, in cases where the medical device is a contact lens, one example is a means for hermetically sealing the container using, as a container for housing the medical device, a common lens case accessory to care items for a contact lens.

After the above-described heating step, the obtained coated medical device obtained may be further subjected to other treatments. Examples of such other treatments include: a method in which the coated medical device is subjected to a similar heating treatment with a buffering agent solution containing no hydrophilic polymer: radiation of an ion beam, electron beam, positron beam, X-rays, γ-rays, neutron rays, or the like; an LbL treatment (a Layer by Layer treatment, for example, a treatment described in WO2013/024800) in which different polymer materials having opposite charges are alternately applied layer by layer; or a crosslinking treatment with a metal ion or a chemical crosslinking treatment (for example, a treatment described in JP2014-533381W).

In addition, before the contacting step and/or the heating step, the surface of the medical device may be pretreated. Examples of the pretreatment include a hydrolysis treatment with an acid such as polyacrylic acid, or an alkali such as sodium hydroxide.

For a method of manufacturing a coated medical device according to the present invention, it is required that the pH of the solution a after the heating step be 6.1 to 8.0. Having the pH within this range eliminates the necessity to wash, with a neutral solution, the resulting coated medical device after the heating step, and has an industrially important meaning from the viewpoint of reducing the manufacturing processes. In some of the cases where the heating step is followed by washing with a neutral solution, the resulting coated medical device needs to be sterilized again. The pH of the solution a after the heating step is preferably 6.5 or more, more preferably 6.6 or more, still more preferably 6.7 or more, particularly preferably 6.8 or more. In addition, the pH is preferably 7.9 or less, more preferably 7.8 or less, still more preferably 7.6 or less.

The pH of the solution a before the heating step is preferably 6.1 to 8.0. The pH of the solution a is preferably 6.5 or more, more preferably 6.6 or more, still more preferably 6.7 or more, particularly preferably 6.8 or more. In addition, the pH is preferably 7.9 or less, more preferably 7.8 or less, still more preferably 7.6 or less. Here, the pH of the solution a before the heating step refers to a pH value measured after the solution a is prepared, and then stirred with a rotor at room temperature (20 to 23°C) for 30 minutes to thereby make the solution uniform.

The pH of the solution a can be measured using a pH meter (for example, a pH meter Eutech pH 2700 (manufactured by Eutech Instruments)). The pH value is rounded off to one decimal place.

There is preferably no covalent bond present between the coating layer of the resulting coated medical device, that is, the hydrophilic polymer layer, and the medical device. Having no such covalent bond makes it possible to manufacture the coated medical device in a simpler step independently of whether the medical device is a hydrous material or a low water content material. Here, having no covalent bond refers to having no chemically reactive group between the hydrophilic polymer layer and the medical device or no group generated by the reaction of the reactive group. Having no chemically reactive group or no group generated by the reaction of the reactive group can be verified by elemental analysis such as electron energy-loss spectroscopy, energy dispersive X-ray spectroscopy, or time-of-flight secondary ion mass spectrometry, or by a composition analysis means. Examples of the chemically reactive group include an azetidinium group, epoxy group, isocyanate group, aziridine group, and azlactone group.

The thickness of the hydrophilic polymer layer is preferably 1 to 99 nm, as observed in a cross section vertical to the longitudinal direction of the coated medical device in a frozen state, using a transmission electron microscope. Having the thickness of the hydrophilic polymer layer within this range makes it easier to exhibit functions such as hydrophilicity and lubricity. The thickness of the hydrophilic polymer layer is more preferably 5 nm or more, still more preferably 10 nm or more. In addition, the thickness of the hydrophilic polymer layer is more preferably 95 nm or less, still more preferably 90 nm or less, still more preferably 85 nm or less, still more preferably 50 nm or less, still more preferably 40 nm or less, still more preferably 30 nm or less, still more preferably 20 nm or less, particularly preferably 15 nm or less. **In** cases where the coated medical device is an ophthalmic lens, the hydrophilic polymer layer having a thickness of less than 100 nm prevents the refraction of light for focusing on the retina from being disturbed, and is less prone to cause poor visibility.

The coating layer of the resulting coated medical device may exist only on a part of the surface of the medical device, may exist on the whole of only one of the surface and the rear face, or may exist on all of the faces.

**In** a coated medical device according to the present invention, at least a part of the hydrophilic polymer layer and the medical device preferably exist in a mixed state to achieve firmer coating. Here, the state in which the hydrophilic polymer layer and the medical device are mixed refers to a state in which an element derived from the medical device is detected in the hydrophilic polymer layer. Whether an element derived from the medical device is detected in the hydrophilic polymer layer can be verified by observing a cross section of the coated medical device by elemental analysis such as scanning transmission electron microscopy, electron energy-loss spectroscopy, energy dispersive X-ray spectroscopy, or time-of-flight secondary ion mass spectrometry, or by a composition analysis means.

Owing to allowing the hydrophilic polymer to be firmly bonded to the surface of the medical device, it is preferable to have a mixed layer in the surface of the medical device, in which mixed layer at least a part of the hydrophilic polymer layer and the medical device are mixed. In cases where the mixed layer exists, it is preferable that a two-layer structure composed of the mixed layer and a single layer made only of a hydrophilic polymer is observed in the hydrophilic polymer layer. The thickness of the mixed layer can be measured, in the same manner as the above-described thickness of the hydrophilic polymer layer, by observing a cross section vertical to the longitudinal direction of the coated medical device in a frozen state, using a transmission electron microscope. To firmly bond the hydrophilic polymer to the surface of the medical device, the thickness of the mixed layer is preferably 3% or more, more preferably 5% or more, still more preferably 10% or more, with respect to the total thickness of the mixed layer and the single layer. In addition, for the hydrophilicity possessed by the hydrophilic polymer to be sufficiently exhibited, the thickness of the mixed layer is preferably 98% or less, more preferably 95% or less, still more preferably 90% or less, particularly preferably 80% or less, with respect to the total thickness of the mixed layer and the single layer.

In addition to the mixed layer on the surface of the medical device, the mixed portion is preferably observed. As used herein, the "mixed portion" refers to a site having no layer structure, in which site the hydrophilic polymer and the medical device are mixed. That is, that the "mixed portion is observed" refers to a state in which a site having no layer structure is observed in a portion other than the mixed layer on the surface of the medical device, and both of an element derived from the hydrophilic polymer and an element derived from the medical device are detected at that site. The thickness of the mixed portion is preferably 3% or more, more preferably 5% or more, still more preferably 10% or more, with respect to the total thickness of the mixed layer and the mixed portion. In addition, for the hydrophilicity possessed by the hydrophilic polymer to be sufficiently exhibited, the thickness of the mixed portion is preferably 98% or less, more preferably 95% or less, still more preferably 90% or less, particularly preferably 80% or less, with respect to the total thickness of the mixed layer and the mixed portion.

In cases where the coated medical device is an ophthalmic lens or the like, the liquid film retention time on the surface of the coated medical device is preferably longer from the viewpoint of not only preventing attachment to the cornea of a wearer but also making it less likely to feel dry and making it possible to maintain satisfactory comfort for a long time.

Here, the liquid film retention time on the surface of the coated medical device refers to a time during which a liquid film on the surface is retained without being broken, as the coated medical device is stationarily immersed in a solution at room temperature (20 to 23°C), pulled up from the solution, and retained in the air in such a manner that the longitudinal direction of the device is the gravitational direction. In cases where a test piece is in the shape of a spherical crown such as a contact lens, the test piece is retained in such a manner that the diametric direction of a circle formed by the brim portion of the spherical crown is the gravitational direction. The expression "the liquid film being broken" refers to a phenomenon in which part of the solution covering the surface of the coated medical device is repelled, leaving the surface of the coated medical device no longer covered wholly with the liquid film. The liquid film retention time is preferably 300 seconds or less, more preferably 200 seconds or less, because the liquid film retention time, if too long, causes moisture to be more easily evaporated from the surface of the coated medical device, thus causing the hydrophilic effect to be low.

In cases where the coated medical device is an ophthalmic lens or the like, the contact angle of a droplet on the surface of the coated medical device is preferably smaller from the viewpoint of not only preventing attachment to the cornea of a wearer but also making it less likely to feel dry and making it possible to maintain satisfactory comfort for a long time. A method of measuring the contact angle of a droplet will be described later. In cases where the medical device is a material having a silicon atom, the contact angle of a droplet is preferably 80° or less, preferably 70° or less, more preferably 65° or less.

In cases where the medical device is a material having no silicon atom, the contact angle of a droplet is preferably 70° or less, more preferably 60° or less, still more preferably 55° or less.

In addition, it can be said that a system in which the rate of change caused by scrubbing in the contact angle of a droplet was smaller allowed a hydrophilic polymer layer having a higher adsorptive force and an excellent durability to be formed on the surface of the medical device. That is, attention is paid to a difference between the contact angle of a droplet after 24-hour immersion in a new phosphate buffer solution (contact angle X of droplet) and the contact angle of a droplet after additional 1-minute scrubbing (contact angle Y of droplet). A method of measuring will be described later.

In cases where the coated medical device is a coated medical device that is, for example, inserted into a living body when used, the surface of the coated medical device preferably has excellent lubricity. The coefficient of friction, as an indicator that represents lubricity, is preferably 0.300 or less, more preferably 0.200 or less, still more preferably 0.100 or less, still more preferably 0.080 or less. On the other hand, the lubricity, if extremely high, makes it difficult in some cases to handle the coated medical device, and thus, the coefficient of friction is preferably 0.001 or more, more preferably 0.002 or more. A method of measuring the coefficient of friction will be described later.

In addition, it can be said that a system in which the rate of change caused by scrubbing in the coefficient of friction was smaller allowed a hydrophilic polymer layer having a higher adsorptive force and an excellent durability to be formed on the surface of the medical device. That is, attention is paid to a difference between the coefficient of friction after 24-hour immersion in a new phosphate buffer solution (coefficient X of friction) and the coefficient of friction after additional 1-minute scrubbing (coefficient Y of friction). A method of measuring will be described later.

In cases where the coated medical device is a soft medical device such as an ophthalmic lens, the tensile elastic modulus is preferably 10.00 MPa or less, more preferably 5.00 MPa or less, still more preferably 3.00 MPa or less, still more preferably 2.00 MPa or less, still more preferably 1.00 MPa or less, particularly preferably 0.60 MPa or less, to enhance the comfort. On the other hand, for easier handling, the tensile elastic modulus of the coated medical device is preferably 0.01 MPa or more, more preferably 0.10 MPa or more, still more preferably 0.20 MPa or more, particularly preferably 0.25 MPa or more. In addition, when the tensile elastic modulus of the medical device before the contacting step and the heating step, and the elastic modulus of the medical device after the contacting step and the heating step are measured, the rate of change in the tensile elastic modulus of the medical device between before and after the contacting step and the heating step, that is, between before and after the coating is preferably ±15.00% or less, more preferably ±13.00% or less, still more preferably ±10.00% or less, to inhibit deformation and the risk of poor feeling of use. A method of measuring the tensile elastic modulus will be described later.

The amount of lipid adhesion on the coated medical device is preferably smaller to enhance the feeling of use, and decrease the risk of bacterial propagation. A method of measuring the amount of lipid adhesion will be described later.

In cases where the coated medical device is an ophthalmic lens, the rate of change in the moisture content of the medical device between before and after the coating is preferably 10 percentage point or less, more preferably 8 percentage point or less, still more preferably 6 percentage point or less, to prevent poor visibility and deformation that are induced by distortion of the refractive index on account of an increase in the moisture content. A method of measuring the moisture content will be described later.

In cases where the coated medical device is an ophthalmic lens, the rate of change in the size of the medical device between before and after the coating is preferably ±5.00% or less, more preferably ±4.00% or less, still more preferably ±3.00% or less, to prevent the cornea from being damaged owing to the deformation. Examples

The present invention will now be described more specifically by way of Examples and the like, but the present invention is not limited to these Examples. First, analytical methods and evaluation methods in Examples and the like will be shown.

### <Hydrophilicity (Liquid Film Retention Time)>

After the heating step, a coated medical device (or medical device) left to stand until cooled to room temperature (20 to 23°C) was pulled up from a solution in a container, and retained in the air in such a manner that the longitudinal direction of the device was the gravitational direction. The time from the point of time when the device started being retained in the air to the time when part of the liquid film covering the surface of the device was broken was measured through visual observation, and the average of the N = 3 values was rated on the following criteria. The maximum of the measurement values was set at 120 seconds.
A: the liquid film on the surface was retained for 20 seconds or more.
B: the liquid film on the surface was broken in 15 seconds or more and less than 20 seconds.
C: the liquid film on the surface was broken in 10 seconds or more and less than 15 seconds.
D: the liquid film on the surface was broken in 1 second or more and less than 10 seconds.
E: the liquid film on the surface was broken in less than 1 second.

### <Hydrophilicity after 24-Hour Immersion in New Phosphate Buffer Solution (Liquid Film Retention Time after 24 Hours)>

To remove the influence of the hydrophilic polymer not sufficiently adsorbed on the surface of the medical device, the coated medical device or the medical device after the heating step was left to stand in 4 mL of a new phosphate buffer solution in a glass vial at room temperature (20 to 23°C) for 24 hours. Then, the coated medical device or the medical device pulled up from the phosphate buffer solution in the glass vial was used as a sample (hereinafter referred to as the "sample S") for evaluation in the same manner as in the above-described "Hydrophilicity".

### <Contact Angle of Droplet after 24-Hour Immersion in New Phosphate Buffer Solution (Contact Angle X of Droplet)>

The sample S was provided separately. A contact angle meter (a liquid droplet method) Drop master DM500 (manufactured by Kyowa Interface Science Co., Ltd.) was used for the measurement of a contact angle of a droplet. Specifically, water was wiped off the surface of the coated medical device (or the medical device), which was then placed on a hemispherical polypropylene having a diameter of 14 mm. The resulting piece was used as a sample. The sample was set in the contact angle meter, a phosphate buffer solution was dropped onto the sample, and the contact angle of the droplet was measured. The amount of the phosphate buffer solution dropped was 20 µL. The average of the N = 3 values was regarded as the contact angle of the droplet.

### <Contact Angle of Droplet after 24-Hour Immersion in New Phosphate Buffer Solution and Additional 1-minute Scrubbing (Contact Angle Y of Droplet)>

The sample S was provided separately, and the sample S was scrubbed between the fingers (the thumb and the index finger) for 1 minute, and evaluated in the same manner as in the above-described "Contact Angle X of Droplet".

### <Moisture Content of Medical Device and Coated Medical Device>

A medical device before being used in a manufacturing method according to the present invention was immersed in a phosphate buffer solution, and left to stand at room temperature (20 to 23°C) for 24 hours or more. Then, the medical device was pulled up from the phosphate buffer solution, the surface moisture was wiped off with a wiping cloth "Kimwipes" (registered trademark) (manufactured by Nippon Paper Crecia Co., Ltd.), and the mass (Ww) of the medical device was measured. Then, the medical device was dried at 40°C for 2 hours in a vacuum dryer and the mass (Wd) of the medical device was measured. From these masses, the moisture content of the medical device was calculated in accordance with the following formula (1). In cases where a value obtained was less than 1%, the value was rated as not higher than the measurement limit, and denoted by "less than 1%". The average of the N = 3 values was regarded as the moisture content. Also with the coated medical device obtained after the heating step, the moisture content was calculated in the same manner. Moisture content (% by mass) of medical device = 100 × (Ww - Wd) / Ww

### <Rate of Change in Moisture Content of Medical Device between before and after Coating>

From the measurement results of the moisture contents of the medical device and the coated medical device, the rate of change in the moisture content was calculated by the following formula (2). Rate (percentage point) of change in moisture content of medical device between before and after coating = moisture content (% by mass) of coated medical device - moisture content (% by mass) of medical device

### <Coefficient of Friction>

Under the below-described conditions, the coefficient of friction of the surface of the coated medical device (or medical device) was measured at N = 5, in which the surface was wet with a phosphate buffer solution. The average of the N = 5 values was regarded as the coefficient of friction.
Apparatus: Friction tester KES-SE (manufactured by Kato Tech Co., Ltd.)
Friction SENS: H
Measurement SPEED: 2 × 1 mm/second
Friction load: 44 g.

### <Coefficient of Friction after 24-Hour Immersion in New Phosphate Buffer Solution (Coefficient X of Friction)>

The sample S was provided separately, and evaluated in the same manner as in the above-described "Coefficient of Friction".

### <Coefficient of Friction after 24-Hour Immersion in New Phosphate Buffer Solution and Additional 1-minute Scrubbing (Coefficient Y of Friction)>

The sample S was provided separately, scrubbed between the fingers (the thumb and the index finger) for 1 minute, and evaluated in the same manner as in the above-described "Coefficient of Friction".

### <Amount of Lipid Adhesion>

In a 20 cc screw tube, 0.03 g of methyl palmitate, 10 g of pure water, and the coated medical device (or medical device) were placed. The screw tube was shaken for 3 hours under the conditions at 37°C and 165 rpm. After shaking, the coated medical device (or medical device) in the screw tube was scrubbed with tap water at 40°C and a household liquid detergent "Mama Lemon" (registered trademark) (manufactured by Lion Corporation). The sample washed was placed in a screw tube containing a phosphate buffer solution, and stored in a refrigerator at 4°C for 24 hours. Then, the coated medical device (or medical device) was visually observed. If any turbid portion was found, the portion was rated as having methyl palmitate adhering therein, and the area of the portion having methyl palmitate adhering therein was observed with respect to the entire surface of the sample.

### <Tensile Elastic Modulus>

A test piece having a width (minimum part) of 5 mm and a length of 14 mm was cut out from a coated medical device (or medical device) using a prescribed punching die. On the test piece, a tensile test was performed using Tensilon Model RTG-1210 (manufactured by A&D Company, Limited). A pulling rate was 100 mm/minute, and a grip-to-grip distance (initial) was 5 mm. A measurement was made on both the medical device before the contacting step and the heating step and the coated medical device after the contacting step and the heating step. The measurement was made at N = 8, and the average of the N = 6 values excluding the maximum value and the minimum value was regarded as the tensile elastic modulus.

### <Rate of Change in Tensile Elastic Modulus of Medical Device between before and after Coating>

From the measurement results of the tensile elastic modulus, the rate of change in the tensile elastic modulus of the medical device between before and after the coating was calculated in accordance with the following formula (3). Rate (%) of change in tensile elastic modulus of medical device between before and after coating = (tensile elastic modulus of coated medical device after coating - tensile elastic modulus of medical device before coating) / tensile elastic modulus of medical device before coating × 100

### <Size>

The longitudinal length of the coated medical device (or medical device) (or the diameter in the case of a circular contact lens or the like) was measured at N = 3, and the average of the N = 3 values was regarded as the size.

### <Rate of Change in Size of Medical Device between before and after Coating>

From the measurement results of the size, the rate of change in the size between before and after the coating was calculated in accordance with the following formula (4). Rate (%) of change in size between before and after coating = (size of coated medical device after coating - size of medical device before coating) / size of medical device before coating × 100

### <Measurement of Weight-average Molecular Weight>

The weight-average molecular weight (hereinafter referred to as "Mw") of a polymer was measured under the following conditions.
Apparatus: Prominence GPC system (manufactured by Shimadzu Corporation)
Pump: LC-20AD
Autosampler: SIL-20AHT
Column oven: CTO-20A
Detector: RID-10A
Column: GMPWXL (manufactured by Tosoh Corporation, 7.8 mm in inner diameter × 30 cm, 13 µm in particle diameter)
Solvent: water/methanol = 1/1 (0.1 N lithium nitrate is added)
Flow rate: 0.5 mL/minute
Measurement time: 30 minutes
Sample concentration: 0.1 to 0.3% by mass
Sample injection amount: 100 µL
Standard sample: Polyethylene oxide standard sample (manufactured by Agilent Technologies, Inc., 0.1 kD to 1258 kD)

### <pH>

The pH of the solution was measured using a pH meter Eutech pH 2700 (manufactured by Eutech Instruments Pte Ltd). The "pH after the heating step" of the solution a was measured immediately after the solution a was cooled to room temperature (20 to 23°C) after the heating step was performed.

### <Thickness of Hydrophilic Polymer Layer>

The coated medical device (or medical device) was immersed in a phosphate buffer solution, and left to stand and washed at room temperature (20 to 23°C) for 24 hours or more. Then, a cross section vertical to the longitudinal direction of the frozen coated medical device (or medical device) was observed using an atomic resolution analytical electron microscope JEM-ARM200F (manufactured by JEOL Ltd.). Measurements, the positions of which were varied, were made at N = 3 at an acceleration voltage of 200 kV. The average of the N = 3 values was regarded as the thickness of the hydrophilic polymer layer. Here, a measurement sample was produced by a method of RuO4 staining, freezing, and ultrathin slicing.

### <Elemental Composition Analysis of Hydrophilic Polymer>

As for the hydrophilic polymer layer of the coated medical device, whether the hydrophilic polymer layer and the medical device are in a mixed state was determined by analyzing the surface of the coated medical device (or medical device) in a dry state after immersion in a phosphate buffer solution, and still standing and washing at room temperature (20 to 23°C) for 24 hours or more by time-of-flight secondary ion mass spectrometry.
Apparatus: TOF.SIMS5 (manufactured by ION-TOF GmbH)
Observational conditions:
   Primary ion: Bi³⁺⁺
   Polarity of secondary ion: positive
   Etching ion: Ar-GCIB (gas cluster ion beam).

### <Phosphate Buffer Solution>

The composition of the phosphate buffer solution used in each of Examples and Comparative Examples is as follows.
KCl: 0.2 g/L
KH₂PO₄: 0.2 g/L
NaCl: 8.0 g/L
Na₂HPO₄: 1.19 g/L
EDTA2Na (disodium dihydrogen ethylenediaminetetraacetate): 0.5 g/L.

### [Synthesis Example 1]

Into a 300mL four-neck flask equipped with a stirrer, a thermometer, a cooling tube, and a three-way cock, 114.08 g of distilled water, 21.62 g of polyethylene glycol with hydrogen-terminated side chain (manufactured by NOF CORPORATION, "BLEMMER" (registered trademark) AE-200; with 4.5 polyethylene glycol chains (in formula (I) m = 4.5 (average value disclosed by the manufacturer), R¹ = hydrogen, X = oxygen, 0.080 mol) and 11.90 g of N,N-dimethylacrylamide (manufactured by KJ Chemicals Corporation, 0.120 mol) were placed, and ultrasonic degassing and nitrogen replacement were performed five times. After nitrogen replacement in the system was completed, the lower part of the flask was immersed in an oil bath set at 90°C with stirring. From the moment the temperature in the flask exceeded 70°C, 27.1 mg of polymerization initiator V-50 (FUJIFILM Wako Pure Chemical Corporation, 0.0999 mmol) dissolved in 20.00 g of distilled water was added in six portions at 5-minute intervals. After the entire amount of the polymerization initiator was added, the flask was kept at an internal temperature of 70 to 75°C for 4 hours to obtain an aqueous solution of the polyethylene glycol monoacrylate with hydrogen-terminated side chain with 4.5 polyethylene glycol chains/N,N-dimethylacrylamide copolymer (copolymerized at a molar ratio of 4/6; Mw, 250,000).

### [Synthesis Example 2]

The polymer was prepared in the same manner as in Synthesis Example 1, except that, as for amounts of materials charged, 73.00 g of distilled water, 5.41 g of polyethylene glycol monoacrylate with hydrogen-terminated side chain (BLEMMER (registered trademark) AE-200, 0.020 mol), 17.84 g (0.180 mol) of N,N-dimethylacrylamide were used to obtain an aqueous solution of the polyethylene glycol monoacrylate with hydrogen-terminated side chain with 4.5 polyethylene glycol chains/N,N-dimethylacrylamide copolymer (copolymerized at a molar ratio of 1/9, Mw, 250,000).

### [Synthesis Example 3]

Into a 100mL three-neck flask, 3.20 g of methoxytetraethylene glycol methacrylate (manufactured by NOF CORPORATION, "BLEMMER" (registered trademark) PME-200; in formula (II), a = 0, k = 2, b = 4, R¹ = a methyl group, X = oxygen, Y = a methyl group; 11.6 mmol), 50.1 mg of 2,2'-azobis[2- (2-imidazolin-2-yl) propane] (manufactured by FUJIFILM Wako Pure Chemical Corporation, VA-061, 0.200 mmol) as a polymerization initiator, and 28.23 g of distilled water (manufactured by FUJIFILM Wako Pure Chemical Corporation) were placed, and a digital thermometer, a Dimroth condenser equipped with a three-way cock, and a sealer equipped with a stirring blade were attached to the flask. After aspirated to 10 mmHg under ultrasonic irradiation, the mixture was flushed with nitrogen. This cycle was repeated five times to remove oxygen dissolved in the mixed solution. Subsequently, the lower part of the flask was immersed in an oil bath, and the mixture was reacted at 60°C for 7 hours with stirring, and then the reaction vessel was removed from the oil bath and air-cooled. Fifty milliliters of methanol was added to the polymerization reaction solution, and the mixture was stirred to reduce the viscosity, and then the mixture was transferred to a 1 L "Teflon" (registered trademark) beaker and dried overnight at 40°C in a vacuum dryer. After drying, 5 mL of isopropanol (IPA) was added to the resulting viscous solid to dissolve it and allowed to stand overnight in a refrigerator. After cooling, the supernatant was removed by decantation and dried in a vacuum dryer at 30°C for 2 hours to obtain a polymer as white powder. The Mw of the resulting methoxypolyethylene glycol methacrylate homoopolymer (number of ethylene oxide repeat units, 4) was 250,000.

### [Reference Example 1]

28.0 parts by mass of polydimethylsiloxane having methacryloyl groups at both ends of the formula (M1) (manufactured by JNC CORPORATION, FM7726, Mw: 30,000), 7.0 parts by mass of silicone monomer (manufactured by JNC CORPORATION, FM0721, Mn: 5,000)) of the formula (M2), 57.9 parts by mass of trifluoroethyl acrylate (manufactured by Osaka Organic Chemical Industry Ltd., "Viscoat" (registered trademark) 3F), 7.0 parts by mass of 2-ethylhexyl acrylate (manufactured by Tokyo Chemical Industry Co., Ltd.), and 0.1 parts by mass of dimethylaminoethyl acrylate (manufactured by Kohjin Co., Ltd.), and, based on the total mass of the monomers, 5,000 ppm of photoinitiator "Irgacure" (registered trademark) 819 (manufactured by NAGASE & CO., LTD.), 5,000 ppm of an ultraviolet absorber (manufactured by Otsuka Chemical Co., Ltd, RUVA-93), and 100 ppm of a colorant (manufactured by Arran Chemical Company Limited, RB246) were prepared. Further, based on 100.0 parts by mass of the total mass of the above-described monomers, 10.0 parts by mass of t-amyl alcohol was prepared, and all of these were mixed and stirred. The stirred mixture was filtered through a membrane filter (pore size: 0.45 µm) to remove insoluble matter, thereby obtaining a monomer mixture.

The monomer mixture was injected into a contact lens mold made of transparent resin (base curved side material, polypropylene; front curved side material, polypropylene), and polymerized by irradiating with light (wavelength, 405 nm (±5 nm); illuminance: 0 to 0.7 mW/cm², 30 minutes).

After polymerization, the obtained molded product together with the mold from which the front curve and base curve had been released was immersed in a 100% by mass aqueous isopropanol solution at 60°C for 1.5 hours, and the contact lens-shaped molded product was peeled off from the mold. The molded body thus obtained was immersed in a large excess of 100% by mass aqueous isopropanol kept at 60°C for 2 hours to extract the impurities such as residual monomers. Thereafter, the product was dried at room temperature (23°C) for 12 hours.

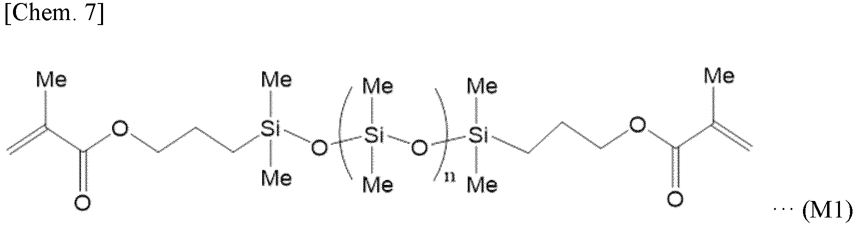

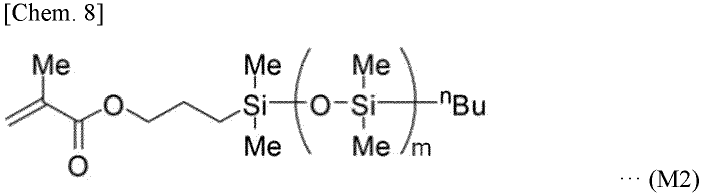

### [Example 1]

As the "medical device", a commercially available silicone hydrogel lens "Acuvue Oasys" (registered trademark) (senofilcon A, manufactured by Johnson & Johnson) containing polyvinylpyrrolidone and silicone as main components was used. As the "container", a glass vial was used. As the "solution a", 3 mL of a solution was used, in which solution a polyethylene glycol monoacrylate with hydrogen-terminated side chain ("BLEMMER" (registered trademark) AE-200)/N,N-dimethylacrylamide copolymer (copolymerized at a molar ratio of 4/6; Mw, 250,000) was contained at 0.10% by mass as the hydrophilic polymer A obtained in the above-described Synthesis Example 1 in a phosphate buffer solution. The medical device was immersed in the solution a ((A) the contacting step), the glass vial was hermetically sealed with a cap ((B) the hermetical sealing step), and the medical device was heated using an autoclave at 121°C for 30 minutes ((C) the heating step). The results of evaluating the resulting coated medical device by the above-described methods are shown in Tables 1 to 3.

### [Example 2]

The same operation as in Example 1 was performed except that, as the solution a, 3 mL of a solution was used, in which solution a polyethylene glycol monoacrylate with hydrogen-terminated side chain ("BLEMMER" (registered trademark) AE-200)/N,N-dimethylacrylamide copolymer (copolymerized at a molar ratio of 1/9; Mw, 150,000) obtained in the above-described Synthesis Example 2 was contained at 0.30% by mass in a phosphate buffer solution. The results of evaluating the resulting coated medical device by the above-described methods are shown in Tables 1 to 3.

### [Example 3]

The same operation as in Example 1 was performed except that, as the solution a, 3 mL of a solution was used, in which solution a polyethylene glycol monoacrylate ("BLEMMER" (registered trademark) AP-400; in formula (II), a = 0, k = 3, b = 6 (average value disclosed by the manufacturer), R¹ = hydrogen, X = oxygen, Y = hydrogen)/N,N-dimethylacrylamide copolymer (copolymerized at a molar ratio of 4/6, Mw: 51,000, manufactured by Osaka Organic Chemical Industry Ltd.) was contained at 0.60% by mass in a phosphate buffer solution. The results of evaluating the resulting medical device by the above-described methods are shown in Tables 1 to 3.

### [Example 4]

The same operation was performed as in Example 1 except that, as the medical device, a commercially available hydrogel lens "MEDALIST 1DAY PLUS" (registered trademark) (hilafilcon B, manufactured by Bausch & Lomb Incorporated) containing 2-hydroxyethyl methacrylate as a main component was used. The results of evaluating the resulting coated medical device by the above-described methods are shown in Tables 1 to 3.

### [Example 5]

The same operation was performed as in Example 2 except that, as the medical device, a commercially available hydrogel lens "MEDALIST 1DAY PLUS" (registered trademark) was used. The results of evaluating the resulting coated medical device by the above-described methods are shown in Tables 1 to 3.

### [Example 6]

The same operation as in Example 1 was performed except that, as the solution a, 3 mL of a solution was used, in which solution a polyethylene glycol polypropylene glycol monoacrylate (manufactured by NOF CORPORATION, "BLEMMER" (registered trademark) 50PEP-300; in formula (II), n = 2, a = 3.5, k = 3, b = 2.5, R¹ = a methyl group, X = oxygen, Y = hydrogen)/N,N-dimethylacrylamide copolymer (copolymerized at a molar ratio of 4/6, Mw: 117,000, manufactured by Osaka Organic Chemical Industry Ltd.) was contained at 0.60% by mass in a phosphate buffer solution. The results of evaluating the resulting coated medical device by the above-described methods are shown in Tables 1 to 3.

### [Example 7]

The same operation was performed as in Example 3 except that, as the medical device, a commercially available hydrogel lens "MEDALIST 1DAY PLUS" (registered trademark) was used. The results of evaluating the resulting coated medical device by the above-described methods are shown in Tables 1 to 3.

### [Example 8]

The same operation was performed as in Example 6 except that, as the medical device, a commercially available hydrogel lens "MEDALIST 1DAY PLUS" (registered trademark) was used. The results of evaluating the resulting coated medical device by the above-described methods are shown in Tables 1 to 3.

### [Example 9]

The same operation as in Example 1 was performed except that, as the solution a, a solution was used, in which solution a polyethylene glycol monoacrylate with hydrogen-terminated side chain (manufactured by NOF CORPORATION, "BLEMMER" (registered trademark) AE-400; in formula (I), m = 10 (average value disclosed by the manufacturer) R¹ = hydrogen, X = oxygen)/N,N-dimethylacrylamide copolymer (manufactured by Osaka Organic Chemical Industry Ltd., copolymerized at a molar ratio of 4/6, Mw: 75,000) was contained at 0.60% by mass in a phosphate buffer solution. The results of evaluating the resulting coated medical device by the above-described methods are shown in Tables 1 to 3.

### [Example 10]

The same operation was performed as in Example 9 except that, as the medical device, a commercially available hydrogel lens "MEDALIST 1DAY PLUS" (registered trademark) was used. The results of evaluating the resulting coated medical device by the above-described methods are shown in Tables 1 to 3.

### [Example 11]

The same operation as in Example 1 was performed except that, as the medical device, a commercially available hydrogel lens "1 DAY ACUVUE " (registered trademark) (manufactured by Johnson & Johnson, etafilcon A) containing 2-hydroxyethyl methacrylate as a main component was used. The results of evaluating the resulting coated medical device by the above-described methods are shown in Tables 1 to 3.

### [Example 12]

The same operation as in Example 9 was performed except that, as the medical device, a commercially available hydrogel lens "1-DAY ACUVUE" (registered trademark) was used, and that, as the solution A, a solution was used, in which solution a hydrophilic polymer A was contained at 0.60% by mass in a phosphate buffer solution. The results of evaluating the resulting coated medical device by the above-described methods are shown in Tables 1 to 3.

### [Example 13]

The same operation as in Example 1 was performed except that, as the medical device, a commercially available hydrogel lens "Proclear 1 day" (registered trademark) (manufactured by CooperVision Inc., omafilcon A) containing 2-hydroxyethyl methacrylate copolymerized with MPC monomer (2-methacryloyloxyethylphosphorylcholine) as a main component was used. The results of evaluating the resulting coated medical device by the above-described methods are shown in Tables 1 to 3.

### [Example 14]

The same operation as in Example 9 was performed except that, as the medical device, a commercially available hydrogel lens "Proclear 1 day" (registered trademark) was used, and that, as the solution A, a solution was used in which solution a hydrophilic polymer A was contained at 0.60% by mass in a phosphate buffer solution. The results of evaluating the resulting coated medical device by the above-described methods are shown in Tables 1 to 3.

### [Example 15]

The same operation as in Example 1 was performed except that, as the medical device, a commercially available hydrogel color lens "1-DAY ACUVUE DEFINE MOIST" (registered trademark) (manufactured by Johnson & Johnson, etafilcon A) containing 2-hydroxyethyl methacrylate as a main component was used. The results of evaluating the resulting coated medical device by the above-described methods are shown in Tables 1 to 3.

### [Example 16]

The same operation as in Example 9 was performed except that, as the medical device, a commercially available hydrogel color lens "1-DAY ACUVUE DEFINE MOIST" (registered trademark) was used, and that, as the solution A, a solution was used, in which solution the hydrophilic polymer A was contained at 0.70% by mass in a phosphate buffer solution. The results of evaluating the resulting coated medical device by the above-described methods are shown in Tables 1 to 3.

### [Example 17]

The same operation as in Example 1 was performed except that, as the medical device, a commercially available silicone hydrogel lens "1-DAY ACUVUE TruEye" (registered trademark) (manufactured by Johnson & Johnson, narafilcon A) containing polyvinylpyrrolidone and silicone as main components was used. The results of evaluating the resulting coated medical device by the above-described methods are shown in Tables 1 to 3.

### [Example 18]

The same operation as in Example 9 was performed except that, as the medical device, a commercially available silicone hydrogel lens "1-DAY ACUVUE TruEye" (registered trademark) was used, and that, as the solution A, a solution was used, in which solution the hydrophilic polymer A was contained at 0.80% by mass in a phosphate buffer solution. The results of evaluating the resulting coated medical device by the above-described methods are shown in Tables 1 to 3.

### [Example 19]

The same operation as in Example 1 was performed except that, as the medical device, a commercially available hydrogel lens "AIR OPTIX AQUA" (registered trademark) (manufactured by Alcon Japan Ltd., lotrafilcon A) having plasma-treated lens surface and containing silicone as a main component was used. The results of evaluating the resulting coated medical device by the above-described methods are shown in Tables 1 to 3.

### [Example 20]

The same operation as in Example 9 was performed except that, as the medical device, a commercially available silicone hydrogel lens "AIR OPTIX AQUA" (registered trademark) was used, and that, as the solution A, a solution was used, in which solution the hydrophilic polymer A was contained at 0.90% by mass in a phosphate buffer solution. The results of evaluating the resulting coated medical device by the above-described methods are shown in Tables 1 to 3.

### [Example 21]

The same operation as in Example 1 was performed except that, as the medical device, a molded product obtained in Reference Example 1 was used, and that, as the solution A, a solution was used, in which solution the hydrophilic polymer A was contained at 0.60% by mass in a phosphate buffer solution.. The results of evaluating the resulting coated medical device by the above-described methods are shown in Tables 1 to 3.

### [Example 22]

The same operation as in Example 9 was performed except that, as the medical device, a molded product obtained in Reference Example 1 was used, and that, as the solution A, a solution was used, in which solution the hydrophilic polymer A was contained at 5.00% by mass in a phosphate buffer solution. The results of evaluating the resulting coated medical device by the above-described methods are shown in Tables 1 to 3.

### [Table 1]

**Table 1**

| | Medical device | Moisture content of medical device (mass%) | Hydrophilic polymer solution | Hydrophilic polymer A Copolymerization ratio Compound a1/ Compound a2 | pH before heating | pH after heating |
|---|---|---|---|---|---|---|
| Example 1 | Silicone hydrogel lens | 38.0 | 0.10 mass% Polyethylene glycol monoacrylate with hydrogen-terminated side chain (with 4.5 polyethylene glycol chains) /N,N-dimethylacrylamide copolymer | 65/35 | 7.1 | 7.2 |
| Example 2 | Silicone hydrogel lens | 38.0 | 0.30 mass% Polyethylene glycol monoacrylate with hydrogen-terminated side chain (with 4.5 polyethylene glycol chains) /N,N-dimethylacrylamide copolymer | 23/77 | 7.1 | 7.2 |
| Example 3 | Silicone hydrogel lens | 38.0 | 0.60 mass% Polypropylene glycol monoacrylate with hydrogen-terminated side chain (with 6 polyethylene glycol chains) /N,N-dimethylacrylamide copolymer | 74/26 | 7.1 | 7.2 |
| Example 4 | Hydrogel lens | 59.0 | 0.10 mass% Polyethylene glycol monoacrylate with hydrogen-terminated side chain (with 4.5 polyethylene glycol chains) /N,N-dimethylacrylamide copolymer | 65/35 | 7.1 | 7.2 |
| Example 5 | Hydrogel lens | 59.0 | 0.30 mass% Polyethylene glycol monoacrylate with hydrogen-terminated side chain (with 4.5 polyethylene glycol chains) /N,N-dimethylacrylamide copolymer | 23/77 | 7.1 | 7.2 |
| Example 6 | Silicone hydrogel lens | 38.0 | 0.60 mass% Polyethylene glycol polypropylene glycol monoacrylate with hydrogen-terminated side chain (with 3.5 polyethylene glycol chains, 2.5 polypropylene glycol chains)/N,N-dimethylacrylamide copolymer | 72/28 | 7.1 | 7.2 |
| Example 7 | Hydrogel lens | 59.0 | 0.60 mass% Polypropylene glycol monoacrylate with hydrogen-terminated side chain (with 6 polyethylene glycol chains) /N,N-dimethylacrylamide copolymer | 74/26 | 7.1 | 7.2 |
| Example 8 | Hydrogel lens | 59.0 | 0.60 mass% Polyethylene glycol polypropylene glycol monoacrylate with hydrogen-terminated side chain (with 3.5 polyethylene glycol chains, 2.5 polypropylene glycol chains) /N,N-dimethylacrylamide copolymer | 72/28 | 7.1 | 7.2 |
| Example 9 | Silicone hydrogel lens | 38.0 | 0.60 mass% Polyethylene glycol monoacrylate with hydrogen-terminated side chain (with 10 polyethylene glycol chains) /N,N-dimethylacrylamide copolymer | 78/22 | 7.2 | 7.3 |
| Example 10 | Hydrogel lens | 59.0 | 0.60 mass% Polyethylene glycol monoacrylate with hydrogen-terminated side chain (with 10 polyethylene glycol chains) /N,N-dimethylacrylamide copolymer | 78/22 | 7.2 | 7.3 |
| Example 11 | Hydrogel lens | 58.0 | 0.10 mass% Polyethylene glycol monoacrylate with hydrogen-terminated side chain (with 4.5 polyethylene glycol chains) /N,N-dimethylacrylamide copolymer | 65/35 | 7.2 | 7.3 |
| Example 12 | Hydrogel lens | 58.0 | 0.60 mass% Polyethylene glycol monoacrylate with hydrogen-terminated side chain (with 10 polyethylene glycol chains) /N,N-dimethylacrylamide copolymer | 78/22 | 7.1 | 7.3 |
| Example 13 | Hydrogel lens | 62.0 | 0.10 mass% Polyethylene glycol monoacrylate with hydrogen-terminated side chain (with 4.5 polyethylene glycol chains) /N,N-dimethylacrylamide copolymer | 65/35 | 7.1 | 7.2 |
| Example 14 | Hydrogel lens | 62.0 | 0.60 mass% Polyethylene glycol monoacrylate with hydrogen-terminated side chain (with 10 polyethylene glycol chains) /N,N-dimethylacrylamide copolymer | 78/22 | 7.2 | 7.3 |
| Example 15 | Hydrogel lens | 59.0 | 0.10 mass% Polyethylene glycol monoacrylate with hydrogen-terminated side chain (with 4.5 polyethylene glycol chains) /N,N-dimethylacrylamide copolymer | 65/35 | 7.1 | 7.2 |
| Example 16 | Hydrogel lens | 59.0 | 0.70 mass% Polyethylene glycol monoacrylate with hydrogen-terminated side chain (with 10 polyethylene glycol chains) /N,N-dimethylacrylamide copolymer | 78/22 | 7.2 | 7.3 |
| Example 17 | Silicone hydrogel lens | 46.0 | 0.10 mass% Polyethylene glycol monoacrylate with hydrogen-terminated side chain (with 4.5 polyethylene glycol chains) /N,N-dimethylacrylamide copolymer | 65/35 | 7.1 | 7.2 |
| Example 18 | Silicone hydrogel lens | 46.0 | 0.80 mass% Polyethylene glycol monoacrylate with hydrogen-terminated side chain (with 10 polyethylene glycol chains) /N,N-dimethylacrylamide copolymer | 78/22 | 7.2 | 7.3 |
| Example 19 | Silicone hydrogel lens | 33.0 | 0.10 mass% Polyethylene glycol monoacrylate with hydrogen-terminated side chain (with 4.5 polyethylene glycol chains) /N,N-dimethylacrylamide copolymer | 65/35 | 7.1 | 7.2 |
| Example 20 | Silicone hydrogel lens | 33.0 | 0.90 mass% Polyethylene glycol monoacrylate with hydrogen-terminated side chain (with 10 polyethylene glycol chains) /N,N-dimethylacrylamide copolymer | 78/22 | 7.2 | 7.3 |
| Example 21 | Reference Example 1 | < 1% | 0.60 mass% Polyethylene glycol monoacrylate with hydrogen-terminated side chain (with 4.5 polyethylene glycol chains) /N,N-dimethylacrylamide copolymer | 65/35 | 7.1 | 7.2 |
| Example 22 | Reference Example 1 | < 1% | 5.00 mass% Polyethylene glycol monoacrylate with hydrogen-terminated side chain (with 10 polyethylene glycol chains) /N,N-dimethylacrylamide copolymer | 78/22 | 7.3 | 7.4 |

### [Table 2]

**Table 2**

| | Liquid film retention time (sec) | Liquid film retention time after 24 hours (sec) | Contact angle X of droplet ( ° ) | Contact angle Y of droplet ( ° ) | Moisture content of coated medical device (mass%) | Thickness of hydrophilic polymer layer (nm) | Form of presence of hydrophilic polymer | Rate of change in moisture content (percentage point) | Coefficient of friction | Coefficient X of friction | Coefficient Y of friction |
|---|---|---|---|---|---|---|---|---|---|---|---|
| Example 1 | A (120 sec) | A (120 sec) | 100 | 99 | 38.3 | 15 | Mixed layer and mixed portion | 0.3 | 0.053 | 0.205 | 0.305 |
| Example 2 | A (120 sec) | A (120 sec) | 101 | 99 | 38.2 | 17 | Mixed layer and mixed portion | 0.2 | 0.099 | 0.280 | 0.315 |
| Example 3 | A (120 sec) | A (120 sec) | 101 | 100 | 38.1 | 5 | Mixed layer and mixed portion | 0.1 | 0.190 | 0.299 | 0.305 |
| Example 4 | A (120 sec) | A (20 sec) | 57 | 56 | 59.4 | 20 | Mixed layer and mixed portion | 0.4 | 0.020 | 0.018 | 0.011 |
| Example 5 | A (120 sec) | A (25 sec) | 53 | 51 | 59.1 | 25 | Mixed layer and mixed portion | 0.1 | 0.020 | 0.018 | 0.016 |
| Example 6 | A (120 sec) | A (120 sec) | 105 | 103 | 38.1 | 5 | Mixed layer and mixed portion | 0.1 | 0.210 | 0.313 | 0.319 |
| Example 7 | A (120 sec) | C (10 sec) | 64 | 63 | 59.1 | 5 | Mixed layer and mixed portion | 0.1 | 0.180 | 0.212 | 0.250 |
| Example 8 | A (120 sec) | C (10 sec) | 65 | 64 | 59.1 | 5 | Mixed layer and mixed portion | 0.1 | 0.160 | 0.200 | 0.215 |
| Example 9 | A (120 sec) | A (120 sec) | 100 | 99 | 38.4 | 12 | Mixed layer and mixed portion | 0.4 | 0.053 | 0.220 | 0.306 |
| Example 10 | A (120 sec) | A (20 sec) | 55 | 52 | 59.4 | 20 | Mixed layer and mixed portion | 0.4 | 0.020 | 0.019 | 0.016 |
| Example 11 | A (120 sec) | A (21 sec) | 52 | 51 | 58.5 | 25 | Mixed layer and mixed portion | 0.5 | 0.015 | 0.012 | 0.013 |
| Example 12 | A (120 sec) | A (25 sec) | 54 | 53 | 58.4 | 29 | Mixed layer and mixed portion | 0.4 | 0.022 | 0.019 | 0.019 |
| Example 13 | A (120 sec) | A (20 sec) | 51 | 50 | 62.3 | 31 | Mixed layer and mixed portion | 0.3 | 0.028 | 0.025 | 0.021 |
| Example 14 | A (120 sec) | A (23 sec) | 53 | 52 | 62.5 | 30 | Mixed layer and mixed portion | 0.5 | 0.035 | 0.032 | 0.030 |
| Example 15 | A (120 sec) | A (20 sec) | 54 | 53 | 59.6 | 28 | Mixed layer and mixed portion | 0.6 | 0.061 | 0.060 | 0.054 |
| Example 16 | A (120 sec) | A (25 sec) | 51 | 50 | 59.5 | 23 | Mixed layer and mixed portion | 0.5 | 0.069 | 0.067 | 0.061 |
| Example 17 | A (120 sec) | A (120 sec) | 102 | 99 | 46.2 | 15 | Mixed layer and mixed portion | 0.2 | 0.100 | 0.355 | 0.390 |
| Example 18 | A (120 sec) | A (120 sec) | 105 | 103 | 46.3 | 12 | Mixed layer and mixed portion | 0.3 | 0.150 | 0.370 | 0.399 |
| Example 19 | A (120 sec) | A (120 sec) | 110 | 105 | 33.2 | 11 | Mixed layer and mixed portion | 0.2 | 0.180 | 0.400 | 0.410 |
| Example 20 | A (120 sec) | A (120 sec) | 108 | 102 | 33.3 | 14 | Mixed layer and mixed portion | 0.3 | 0.190 | 0.401 | 0.412 |
| Example 21 | D (5 sec) | E (< 1 sec) | 120 | 119 | < 1% | 5 | Mixed layer and mixed portion | - | 0.610 | 0.790 | 0.850 |
| Example 22 | D (5 sec) | E (< 1 sec) | 121 | 120 | < 1% | 5 | Mixed layer and mixed portion | - | 0.650 | 0.780 | 0.856 |

### [Table 3]

**Table 3**

| | Amount of lipid adhesion | Tensile elastic modulus of medical device (MPa) | Tensile elastic modulus of coated medical device (MPa) | Rate of change in tensile elastic modulus (%) | Size of medical device (mm) | Size of coated medical device (mm) | Rate of change in size (%) |
|---|---|---|---|---|---|---|---|
| Example 1 | 5% of total area | 0.70 | 0.72 | 2.90 | 14.20 | 14.19 | -0.07 |
| Example 2 | 5% of total area | 0.70 | 0.71 | 1.40 | 14.20 | 14.21 | 0.07 |
| Example 3 | 5% of total area | 0.70 | 0.72 | 2.90 | 14.20 | 14.21 | 0.07 |
| Example 4 | No adhesion | 0.26 | 0.27 | 3.80 | 14.20 | 14.19 | -0.07 |
| Example 5 | No adhesion | 0.26 | 0.27 | 3.80 | 14.20 | 14.19 | -0.07 |
| Example 6 | 5% of total area | 0.70 | 0.72 | 2.90 | 14.20 | 14.21 | 0.07 |
| Example 7 | No adhesion | 0.26 | 0.27 | 3.80 | 14.20 | 14.21 | 0.07 |
| Example 8 | No adhesion | 0.26 | 0.27 | 3.80 | 14.20 | 14.21 | 0.07 |
| Example 9 | 5% of total area | 0.70 | 0.72 | 2.90 | 14.20 | 14.21 | 0.07 |
| Example 10 | No adhesion | 0.26 | 0.27 | 3.80 | 14.20 | 14.21 | 0.07 |
| Example 11 | No adhesion | 0.30 | 0.28 | -6.80 | 14.20 | 13.90 | -2.10 |
| Example 12 | No adhesion | 0.30 | 0.26 | -13.1 | 14.20 | 13.95 | -1.80 |
| Example 13 | No adhesion | 0.41 | 0.42 | 2.43 | 14.15 | 14.20 | 0.40 |
| Example 14 | No adhesion | 0.41 | 0.43 | 4.87 | 14.15 | 14.21 | 0.42 |
| Example 15 | No adhesion | 0.26 | 0.25 | -3.85 | 14.05 | 14.10 | 0.36 |
| Example 16 | No adhesion | 0.26 | 0.25 | -3.85 | 14.05 | 14.15 | 0.71 |
| Example 17 | 10% of total area | 0.70 | 0.71 | 1.43 | 14.20 | 14.30 | 0.70 |
| Example 18 | 10% of total area | 0.70 | 0.71 | 1.43 | 14.20 | 14.25 | 0.35 |
| Example 19 | 10% of total area | 1.00 | 1.10 | 10 | 14.15 | 14.20 | 0.35 |
| Example 20 | 10% of total area | 1.00 | 1.10 | 10 | 14.15 | 14.21 | 0.42 |
| Example 21 | 80% of total area | 0.53 | 0.52 | -1.89 | 14.00 | 14.01 | 0.07 |
| Example 22 | 80% of total area | 0.53 | 0.52 | -1.89 | 14.00 | 14.02 | 0.14 |

### [Comparative Example 1]

The same operation as in Example 1 was performed except that, in place of the solution a, a solution was used, in which solution an acrylic acid/vinylpyrrolidone/N,N-dimethylacrylamide copolymer (copolymerized at a molar ratio of 1/1/2, Mw: 550,000, manufactured by Osaka Organic Chemical Industry Ltd.) was contained at 0.20% by mass in a phosphate buffer solution. The results of evaluating the resulting coated medical device (no hydrophilic polymer layer was identified) by the above-described methods are shown in Tables 4 to 6.

### [Comparative Example 2]

The same operation as in Example 1 was performed except that, in place of the solution a, a solution was used, in which solution an acrylic acid/vinylpyrrolidone/N,N-dimethylacrylamide copolymer (copolymerized at a molar ratio of 1/1/2, Mw: 330,000, manufactured by Osaka Organic Chemical Industry Ltd.) was contained at 0.10% by mass in a phosphate buffer solution. The results of evaluating the resulting coated medical device (no hydrophilic polymer layer was identified) by the above-described methods are shown in Tables 4 to 6.

### [Comparative Example 3]

The same operation as in Example 1 was performed except that, in place of the solution a, a solution was used, in which solution an acrylic acid/N,N-dimethylacrylamide copolymer (manufactured by Osaka Organic Chemical Industry Ltd., copolymerized at a molar ratio of 1/9, Mw: 300,000) was contained at 0.10% by mass in a phosphate buffer solution. The results of evaluating the resulting coated medical device (no hydrophilic polymer layer was identified) by the above-described methods are shown in Tables 4 to 6.

### [Comparative Example 4]

The same operation was performed as in Example 1 except that, as the medical device, a commercially available hydrogel lens "MEDALIST 1DAY PLUS" (registered trademark) (hilafilcon B, manufactured by Bausch & Lomb Incorporated) was used, and that, in place of the solution a, a solution was used, in which solution polyethylene glycol (manufactured by Wako Pure Chemical Industries, Ltd., Mw: 500,000) was contained at 0.30% by mass in a phosphate buffer solution. The results of evaluating the resulting coated medical device (no hydrophilic polymer layer was identified) by the above-described methods are shown in Tables 4 to 6.

### [Comparative Example 5]

The same operation as in Example 1 was performed except that, in place of the solution a, a solution was used, in which solution an acrylic acid/vinylpyrrolidone copolymer (manufactured by Osaka Organic Chemical Industry Ltd., copolymerized at a molar ratio of 1/4, Mw: 590,000) was contained at 0.10% by mass in a phosphate buffer solution. The results of evaluating the resulting coated medical device (no hydrophilic polymer layer was identified) by the above-described methods are shown in Tables 4 to 6.

### [Comparative Example 6]

The same operation as in Example 1 was performed except that, in place of the solution a, a solution was used, in which solution an acrylic acid/vinylpyrrolidone copolymer (manufactured by Osaka Organic Chemical Industry Ltd., copolymerized at a molar ratio of 1/9, Mw: 390,000) was contained at 0.10% by mass in a phosphate buffer solution. The results of evaluating the resulting coated medical device (no hydrophilic polymer layer was identified) by the above-described methods are shown in Tables 4 to 6.

### [Comparative Example 7]

The same operation as in Example 1 was performed except that, in place of the solution a, a solution was used, in which solution an acrylic acid/2-hydroxyethyl methacrylate/N,N-dimethylacrylamide copolymer (manufactured by Osaka Organic Chemical Industry Ltd., copolymerized at a molar ratio of 1/1/2, Mw: 430,000) was contained at 0.20% by mass in a phosphate buffer solution. The results of evaluating the resulting coated medical device (no hydrophilic polymer layer was identified) by the above-described methods are shown in Tables 4 to 6.

### [Comparative Example 8]

The same operation as in Example 1 was performed except that, in place of the solution a, a solution was used, in which solution an acrylic acid/2-hydroxyethyl methacrylate/N,N-dimethylacrylamide copolymer (manufactured by Osaka Organic Chemical Industry Ltd., copolymerized at a molar ratio of 1/1/8, Mw: 480,000) was contained at 0.20% by mass in a phosphate buffer solution. The results of evaluating the resulting coated medical device (no hydrophilic polymer layer was identified) by the above-described methods are shown in Tables 4 to 6.

### [Comparative Example 9]

The same operation as in Example 1 was performed except that, in place of the solution a, a solution was used, in which solution polyvinylpyrrolidone (manufactured by Osaka Organic Chemical Industry Ltd., Mw: 500,000) was contained at 0.30% by mass in a phosphate buffer solution. The results of evaluating the resulting coated medical device (no hydrophilic polymer layer was identified) by the above-described methods are shown in Tables 4 to 6.

### [Comparative Example 10]

The same operation as in Example 1 was performed except that, in place of the solution a, a solution was used, in which solution poly(N,N-dimethylacrylamide) (manufactured by Wako Pure Chemical Industries, Ltd., Mw: 700,000) was contained at 0.30% by mass in a phosphate buffer solution. The results of evaluating the resulting coated medical device (no hydrophilic polymer layer was identified) by the above-described methods are shown in Tables 4 to 6.

### [Comparative Example 11]

The same operation as in Example 1 was performed except that, in place of the solution a, a phosphate buffer solution was used. The results of evaluating the resulting medical device (no polymer layer was identified) by the above-described methods are shown in Tables 4 to 6.

### [Comparative Example 12]

The same operation was performed as in Comparative Example 11 except that, as the medical device, a commercially available hydrogel lens "MEDALIST 1DAY PLUS" (registered trademark) was used. The results of evaluating the resulting medical device (no polymer layer was identified) by the above-described methods are shown in Tables 4 to 6.

### [Comparative Example 13]

The same operation was performed as in Comparative Example 10 except that, as the medical device, a commercially available hydrogel lens "MEDALIST 1DAY PLUS" (registered trademark) was used. The results of evaluating the resulting medical device (no polymer layer was identified) by the above-described methods are shown in Tables 4 to 6.

### [Comparative Example 14]

The same operation as in Comparative Example 13 was performed except that, in place of the solution a, a solution was used, in which solution a methoxypolyethylene glycol methacrylate homopolymer (with 4 polyethylene glycol chains, Mw: 250,000) obtained in Synthesis Example 3 described above was contained at 0.20% by mass in a phosphate buffer solution. The results of evaluating the resulting coated medical device (no hydrophilic polymer layer was identified) by the above-described methods are shown in Tables 4 to 6.

### [Comparative Example 15]

The same operation as in Comparative Example 13 was performed except that, in place of the solution a, a solution was used, in which solution a methoxypolyethylene glycol methacrylate homopolymer obtained in Synthesis Example 3 described above was contained at 0.20% by mass in a phosphate buffer solution. The results of evaluating the resulting coated medical device (no hydrophilic polymer layer was identified) by the above-described methods are shown in Tables 4 to 6.

### [Comparative Example 16]

The same operation as in Example 1 was performed except that, in place of the solution a, a solution was used, in which solution a methoxypolyethylene glycol methacrylate homopolymer obtained in Synthesis Example 3 described above was contained at 0.20% by mass in a phosphate buffer solution. The results of evaluating the resulting coated medical device (no hydrophilic polymer layer was identified) by the above-described methods are shown in Tables 4 to 6.

### [Comparative Example 17]

The same operation as in Comparative Example 11 was performed except that, as the medical device, a commercially available hydrogel lens "1 DAY ACUVUE" (registered trademark) was used. The results of evaluating the resulting medical device (no polymer layer was identified) by the above-described methods are shown in Tables 4 to 6.

### [Comparative Example 18]

The same operation was performed as in Comparative Example 11 except that, as the medical device, a commercially available hydrogel lens "Proclear 1 day" (registered trademark) was used. The results of evaluating the resulting medical device (no polymer layer was identified) by the above-described methods are shown in Tables 4 to 6.

### [Comparative Example 19]

The same operation was performed as in Comparative Example 11 except that, as the medical device, a commercially available hydrogel color lens "1-DAY ACUVUE DEFINE MOIST" (registered trademark) was used. The results of evaluating the resulting medical device (no polymer layer was identified) by the above-described methods are shown in Tables 4 to 6.

### [Comparative Example 20]

The same operation was performed as in Comparative Example 11 except that, as the medical device, a commercially available silicone hydrogel lens "1-DAY ACUVUE TruEye" (registered trademark) was used. The results of evaluating the resulting medical device (no polymer layer was identified) by the above-described methods are shown in Tables 4 to 6.

### [Comparative Example 21]

The same operation was performed as in Comparative Example 11 except that, as the medical device, a commercially available silicone hydrogel lens "AIR OPTIX AQUA" (registered trademark) was used. The results of evaluating the resulting medical device (no polymer layer was identified) by the above-described methods are shown in Tables 4 to 6.

### [Comparative Example 22]

The same operation as in Comparative Example 11 was performed except that, as the medical device, a molded product obtained in Reference Example 1 was used. The results of evaluating the resulting medical device (no polymer layer was identified) by the above-described methods are shown in Tables 4 to 6.

### [Table 4]

**Table 4**

| | Medical device | Moisture content of medical device (mass%) | Hydrophilic polymer solution | Hydrophilic polymer A Copolymerization ratio Compound a1/ Compound a2 | pH before heating | pH after heating |
|---|---|---|---|---|---|---|
| Comparative Example 1 | Silicone hydrogel lens | 38.0 | 0.20 mass% Acrylic acid/vinylpyrrolidone /N,N-dimethylacrylamide copolymer | 0/81 | 7.0 | 7.0 |
| Comparative Example 2 | Silicone hydrogel lens | 38.0 | 0.10 mass% Acrylic acid/vinylpyrrolidone /N,N-dimethylacrylamide copolymer | 0/81 | 7.0 | 7.1 |
| Comparative Example 3 | Silicone hydrogel lens | 38.0 | 0.10 mass% Acrylic acid /N,N-dimethylacrylamide copolymer | 0/93 | 6.9 | 7.0 |
| Comparative Example 4 | Hydrogel lens | 59.0 | 0.30 mass% Polyethylene glycol | 0/0 | 7.0 | 7.0 |
| Comparative Example 5 | Silicone hydrogel lens | 38.0 | 0.10 mass% Acrylic acid/vinylpyrrolidone copolymer | 0/86 | 6.9 | 7.0 |
| Comparative Example 6 | Silicone hydrogel lens | 38.0 | 0.10 mass% Acrylic acid/vinylpyrrolidone copolymer | 0/93 | 6.9 | 7.0 |
| Comparative Example 7 | Silicone hydrogel lens | 38.0 | 0.20 mass% Acrylic acid/2-hydroxyethyl methacrylate /N,N-dimethylacrylamide copolymer | 0/50 | 6.8 | 6.9 |
| Comparative Example 8 | Silicone hydrogel lens | 38.0 | 0.20 mass% Acrylic acid/2-hydroxyethyl methacrylate /N,N-dimethylacrylamide copolymer | 0/80 | 7.0 | 7.1 |
| Comparative Example 9 | Silicone hydrogel lens | 38.0 | 0.30 mass% Polyvinylpyrrolidone | 0/100 | 7.0 | 7.1 |
| Comparative Example 10 | Silicone hydrogel lens | 38.0 | 0.30 mass% Poly(N,N-dimethylacrylamide) | 0/100 | 7.1 | 7.2 |
| Comparative Example 11 | Silicone hydrogel lens | 38.0 | Absent | N/A | 7.0 | 7.2 |
| Comparative Example 12 | Hydrogel lens | 59.0 | Absent | N/A | 7.0 | 7.1 |
| Comparative Example 13 | Hydrogel lens | 59.0 | 0.30 mass% Poly(N,N-dimethylacrylamide) | 0/100 | 7.1 | 7.2 |
| Comparative Example 14 | Hydrogel lens | 59.0 | 0.20 mass% Methoxypolyethylene glycol methacrylate homopolymer (number of ethylene oxide repeat units, 4) | 100/0 | 7.1 | 7.2 |
| Comparative Example 15 | Hydrogel lens | 59.0 | 0.20 mass% Methoxypolyethylene glycol methacrylate homopolymer (number of ethylene oxide repeat units, 4) | 100/0 | 7.1 | 7.2 |
| Comparative Example 16 | Silicone hydrogel lens | 38.0 | 0.20 mass% Methoxypolyethylene glycol methacrylate homopolymer (number of ethylene oxide repeat units, 4) | 100/0 | 7.1 | 7.2 |
| Comparative Example 17 | Hydrogel lens | 58.0 | Absent | N/A | 7.0 | 7.0 |
| Comparative Example 18 | Hydrogel lens | 60.0 | Absent | N/A | 7.0 | 7.0 |
| Comparative Example 19 | Hydrogel lens | 58.0 | Absent | N/A | 7.0 | 7.0 |
| Comparative Example 20 | Silicone hydrogel lens | 46.0 | Absent | N/A | 7.0 | 7.0 |
| Comparative Example 21 | Silicone hydrogel lens | 33.0 | Absent | N/A | 7.0 | 7.0 |
| Comparative Example 22 | Reference Example 1 | < 1% | Absent | N/A | 7.0 | 7.0 |

### [Table 5]

**Table 5**

| | Liquid film retention time (sec) | Liquid film retention time after 24 hours (sec) | Contact angle X of droplet (°) | Contact angle Y of droplet (°) | Moisture content of coated medical device (mass%) | Thickness of hydrophilic polymer layer (nm) | Form of presence of hydrophilic polymer | Rate of change in moisture content (percentage point) | Coefficient of friction | Coefficient X of friction | Coefficient Y of friction |
|---|---|---|---|---|---|---|---|---|---|---|---|
| Comparative Example 1 | D (1 sec) | D (1 sec) | 101 | 103 | 38.0 | 0 | Layer not observed | 0 | 0.250 | 0.340 | 0.339 |
| Comparative Example 2 | D (1 sec) | D (1 sec) | 104 | 102 | 38.0 | 0 | Layer not observed | 0 | 0.248 | 0.342 | 0.346 |
| Comparative Example 3 | D (1 sec) | D (1 sec) | 103 | 100 | 38.0 | 0 | Layer not observed | 0 | 0.251 | 0.349 | 0.341 |
| Comparative Example 4 | A (94 sec) | D (3 sec) | 75 | 76 | 59.0 | 0 | Layer not observed | 0 | 0.120 | 0.350 | 0.351 |
| Comparative Example 5 | D (1 sec) | D (1 sec) | 106 | 101 | 38.0 | 0 | Layer not observed | 0 | 0.254 | 0.348 | 0.341 |
| Comparative Example 6 | D (1 sec) | D (1 sec) | 105 | 101 | 38.0 | 0 | Layer not observed | 0 | 0.261 | 0.339 | 0.343 |
| Comparative Example 7 | D (1 sec) | D (1 sec) | 104 | 102 | 38.0 | 0 | Layer not observed | 0 | 0.262 | 0.345 | 0.350 |
| Comparative Example 8 | D (1 sec) | D (1 sec) | 106 | 108 | 38.0 | 0 | Layer not observed | 0 | 0.259 | 0.351 | 0.347 |
| Comparative Example 9 | A (120 sec) | D (4 sec) | 99 | 103 | 38.0 | 0 | Layer not observed | 0 | 0.123 | 0.325 | 0.340 |
| Comparative Example 10 | A (120 sec) | D (4 sec) | 98 | 105 | 38.0 | 0 | Layer not observed | 0 | 0.110 | 0.350 | 0.318 |
| Comparative Example 11 | D (7 sec) | D (5 sec) | 107 | 103 | No | 0 | Layer not observed | Not changed | 0.340 | 0.330 | 0.341 |
| Comparative Example 12 | D (9 sec) | D (9 sec) | 77 | 75 | No | 0 | Layer not observed | Not changed | 0.350 | 0.348 | 0.351 |
| Comparative Example 13 | A (120 sec) | D (3 sec) | 75 | 78 | 59.0 | 0 | Layer not observed | 0 | 0.124 | 0.349 | 0.353 |
| Comparative Example 14 | A (120 sec) | D (2 sec) | 76 | 78 | 59.0 | 0 | Layer not observed | 0 | 0.349 | 0.351 | 0.353 |
| Comparative Example 15 | A (120 sec) | D (3 sec) | 75 | 77 | 59.0 | 0 | Layer not observed | 0 | 0.351 | 0.356 | 0.353 |
| Comparative Example 16 | A (120 sec) | A (90 sec) | 101 | 102 | 38.0 | 0 | Layer not observed | 0 | 0.341 | 0.342 | 0.345 |
| Comparative Example 17 | D (3 sec) | D (1 sec) | 78 | 79 | No | 0 | Layer not observed | Not changed | 0.434 | 0.455 | 0.460 |
| Comparative Example 18 | D (3 sec) | D (1 sec) | 80 | 79 | No | 0 | Layer not observed | Not changed | 0.323 | 0.350 | 0.351 |
| Comparative Example 19 | D (3 sec) | D (1 sec) | 81 | 82 | No | 0 | Layer not observed | Not changed | 0.450 | 0.460 | 0.462 |
| Comparative Example 20 | D (3 sec) | D (1 sec) | 105 | 104 | No | 0 | Layer not observed | Not changed | 0.360 | 0.365 | 0.363 |
| Comparative Example 21 | D (2 sec) | D (1 sec) | 101 | 102 | No | 0 | Layer not observed | Not changed | 0.420 | 0.430 | 0.431 |
| Comparative Example 22 | E (<1 sec) | E (< 1 sec) | 119 | 121 | No | 0 | Layer not observed | Not changed | 0.850 | 0.858 | 0.857 |

### [Table 6]

**Table 6**

| | Amount of lipid adhesion | Tensile elastic modulus of medical device (MPa) | Tensile elastic modulus of coated medical device (MPa) | Rate of change in tensile elastic modulus (%) | Size of medical device (mm) | Size of coated medical device (mm) | Rate of change in size (%) |
|---|---|---|---|---|---|---|---|
| Comparative Example 1 | 20% of total area | 0.71 | 0.72 | 1.40 | 14.20 | 14.21 | 0.07 |
| Comparative Example 2 | 20% of total area | 0.71 | 0.71 | 0.56 | 14.20 | 14.21 | 0.07 |
| Comparative Example 3 | 20% of total area | 0.71 | 0.70 | -1.40 | 14.20 | 14.19 | -0.07 |
| Comparative Example 4 | No adhesion | 0.26 | 0.26 | 0.00 | 14.20 | 14.21 | 0.07 |
| Comparative Example 5 | 20% of total area | 0.71 | 0.71 | 0.56 | 14.20 | 14.20 | 0.04 |
| Comparative Example 6 | 20% of total area | 0.71 | 0.70 | -2.40 | 14.20 | 14.20 | 0.04 |
| Comparative Example 7 | 20% of total area | 0.71 | 0.70 | -1.10 | 14.20 | 14.19 | -0.07 |
| Comparative Example 8 | 20% of total area | 0.71 | 0.71 | 0.80 | 14.20 | 14.21 | 0.07 |
| Comparative Example 9 | 20% of total area | 0.71 | 0.71 | 0.90 | 14.20 | 14.21 | 0.07 |
| Comparative Example 10 | 20% of total area | 0.71 | 0.71 | 0.70 | 14.20 | 14.21 | 0.07 |
| Comparative Example 11 | 20% of total area | 0.71 | 0.71 | 0.00 | 14.20 | 14.20 | 0.00 |
| Comparative Example 12 | No adhesion | 0.26 | 0.26 | 0.00 | 14.20 | 14.20 | 0.00 |
| Comparative Example 13 | No adhesion | 0.26 | 0.26 | 0.00 | 14.20 | 14.21 | 0.07 |
| Comparative Example 14 | No adhesion | 0.26 | 0.26 | 0.00 | 14.20 | 14.19 | -0.07 |
| Comparative Example 15 | No adhesion | 0.26 | 0.26 | 0.00 | 14.20 | 14.21 | 0.07 |
| Comparative Example 16 | 20% of total area | 0.71 | 0.72 | 1.40 | 14.20 | 14.21 | 0.07 |
| Comparative Example 17 | No adhesion | 0.30 | N/A | N/A | 14.20 | N/A | N/A |
| Comparative Example 18 | No adhesion | 0.39 | N/A | N/A | 14.20 | N/A | N/A |
| Comparative Example 19 | No adhesion | 0.30 | N/A | N/A | 14.20 | N/A | N/A |
| Comparative Example 20 | 20% of total area | 0.70 | N/A | N/A | 14.20 | N/A | N/A |
| Comparative Example 21 | 20% of total area | 1.10 | N/A | N/A | 14.20 | N/A | N/A |
| Comparative Example 22 | Totally adhered | 0.53 | N/A | N/A | 14.00 | N/A | N/A |

From the comparison of the results between Examples and Comparative Examples, it is evident that the present invention can provide a medical device with not only sufficient hydrophilicity and lubricity but also an excellent lipid adhesion inhibiting capability

In addition, in cases where attention is paid to changes caused by scrubbing in the contact angle of a droplet and in the coefficient of friction, that is, a difference between the contact angle X of a droplet and the contact angle Y of a droplet and a difference between the coefficient X of friction and the coefficient Y of friction, it can be said that a system in which the rate of change was smaller allowed a hydrophilic polymer layer having a higher adsorptive force and an excellent durability to be formed on the surface of the medical device. The results of Examples 4, 5, and 10 to 16 match this consequence.

## Claims

1. A coated medical device comprising a medical device and a hydrophilic polymer layer coating the surface of said medical device; wherein said hydrophilic polymer layer contains a hydrophilic polymer A, said hydrophilic polymer A containing, as monomer units, a compound a1 of the following general formula (I) or the following general formula (II), and a compound a2 having an amide group; and wherein the copolymerization ratio of said compound a1 to said compound a2 is 1/99 to 90/10 by mass; 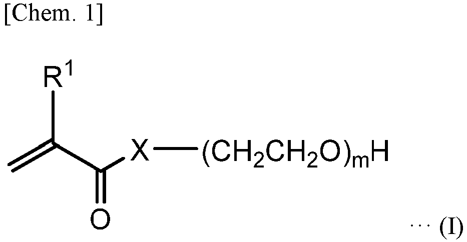
wherein, in the general formula (I), R¹ is a hydrogen atom or a methyl group; X is an oxygen atom or NR², wherein R² is a hydrogen atom or an alkyl group; and m is an integer of 1 to 30; 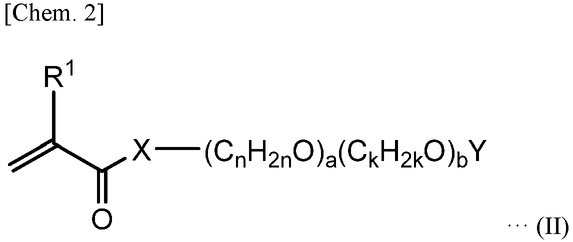
wherein, in the general formula (II), R¹ is a hydrogen atom or a methyl group; X is an oxygen atom or NR², wherein R² is a hydrogen atom or an alkyl group; n is 2 or 4; k is 3 or 4; a is an integer of 0 to 30; b is an integer of 1 to 30; and Y is a hydrogen atom, a methyl group, an ethyl group, a propyl group, an isopropyl group, or a butyl group.

2. The coated medical device according to claim 1, comprising a mixed layer of said hydrophilic polymer layer and said medical device.

3. The coated medical device according to claim 1 or 2, wherein said medical device comprises a material selected from the group consisting of a hydrogel, a silicone hydrogel, a low water content soft material, and a low water content hard material.

4. The coated medical device according to claim 3, wherein said hydrogel is selected from the group consisting of tefilcon, tetrafilcon, helfilcon, mafilcon, polymacon, hioxifilcon, alfafilcon, omafilcon, nelfilcon, nesofilcon, hilafilcon, acofilcon, deltafilcon, etafilcon, focofilcon, ocufilcon, phemfilcon, methafilcon, and vilfilcon.

5. The coated medical device according to claim 3, wherein said silicone hydrogel is selected from the group consisting of lotrafilcon, galyfilcon, narafilcon, senofilcon, comfilcon, enfilcon, balafilcon, efrofilcon, fanfilcon, somofilcon, samfilcon, olifilcon, asmofilcon, formofilcon, stenfilcon, abafilcon, mangofilcon, riofilcon, sifilcon, larafilcon, and delefilcon.

6. The coated medical device according to claim 3, wherein said low water content soft material or said low water content hard material is selected from the group consisting of polysulfone, polystyrene, polymethyl methacrylate, polyurethane, and polyamide.

7. The coated medical device according to any one of claims 1 to 6, wherein said medical device is selected from the group consisting of an ophthalmic lens, a dermal covering material, a wound dressing material, a skin protection material, a skin medicine carrier, an infusion tube, a gas delivery tube, a drainage tube, a blood circuit, a covering tube, a catheter, a stent, a sheath, a biosensor chip, artificial heart and lung, and an endoscopic covering material.

8. A method of manufacturing the coated medical device according to any one of claims 1 to 7, comprising:
(A) a contacting step of housing a medical device in a container, and bringing said medical device into contact with a solution a containing a hydrophilic polymer A; and
(C) a heating step of heating said container,
wherein said hydrophilic polymer A contains, as monomer units, a compound a1 of the following general formula (I) or the following general formula (II) and a compound a2 having an amide group;
wherein the copolymerization ratio of said compound a1 to said compound a2 is 1/99 to 90/10 by mass; 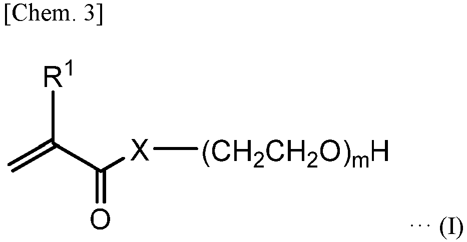
wherein, in the general formula (I), R¹ is a hydrogen atom or a methyl group; X is an oxygen atom or NR², wherein R² is a hydrogen atom or an alkyl group; and m is an integer of 1 to 30; 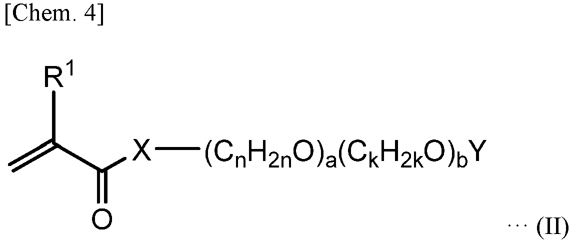
wherein, in the general formula (II), R¹ is a hydrogen atom or a methyl group; X is an oxygen atom or NR², wherein R² is a hydrogen atom or an alkyl group; n is 2 or 4; k is 3 or 4; a is an integer of 0 to 30; b is an integer of 1 to 30; and Y is a hydrogen atom, a methyl group, an ethyl group, a propyl group, an isopropyl group, or a butyl group;
wherein the pH of said solution a after said heating step is 6.1 to 8.0.

9. The method of manufacturing the coated medical device according to claim 8, further comprising (B) a hermetical sealing step of hermetically sealing said container in which said medical device is housed.

10. The method of manufacturing the coated medical device according to claim 8 or 9, wherein said medical device is sterilized by said heating step.
